# EUROPEAN PATENT APPLICATION

(11) **EP 3 305 175 A2**
(43) Date of publication of application: **11.04.2018**
(21) Application number: 17194434.1
(22) Date of filing: 02.10.2017
(51) Int. Cl.: A61B 3/10, A61B 3/00, G06T 7/10

(54) **TOMOGRAPHIC IMAGE ACQUISITION APPARATUS AND TOMOGRAPHIC IMAGE ACQUISITION METHOD**

(30) Priority: 05.10.2016 JP 2016196898; 28.10.2016 JP 2016212125
(71) Applicant: CANON KABUSHIKI KAISHA, Ohta-ku Tokyo 146-8501 (JP)
(72) Inventor: TAKENO, Kohei, Ohta-ku, Tokyo 146-8501 (JP); TANAKA, Hiroyoshi, Ohta-ku, Tokyo (JP)
(74) Representative: Walker, Philip Martin

(57) **Abstract**

A tomographic image acquisition apparatus includes first designation means for designating a first position in which to acquire a first tomographic image in a fundus image of an eye to be examined, first acquisition means for acquiring the first tomographic image corresponding to the first position, second designation means for designating a second position in which to acquire a second tomographic image in the first tomographic image, the second tomographic image having a higher resolution than the first tomographic image, and second acquisition means for acquiring the second tomographic image based on the first position and the second position.

## Description

### BACKGROUND OF THE INVENTION

### Field of the Invention

Aspects of the present invention generally relate to a tomographic image acquisition apparatus and a tomographic image acquisition method, and particularly to a tomographic image acquisition apparatus and a tomographic image acquisition method each of which is capable of efficiently acquiring a high-resolution tomographic image.

### Description of the Related Art

In recent years, a tomographic image acquisition apparatus (an optical coherence tomography (OCT) apparatus) using interference of low coherence light has been put into practical use as an ophthalmic examination apparatus. Furthermore, in OCT, making the beam diameter of measurement light larger has enabled acquiring a tomographic image of the retina with an improved transverse resolution. However, making the beam diameter of measurement light larger has brought about two issues in acquiring a tomographic image of the retina. One issue is a reduction in signal-to-noise (S/N) ratio and transverse resolution due to aberrations of an eye to be examined, and the other issue is a decrease of a region with a high transverse resolution due to a decrease in depth of focus.

To solve the former issue, an AO-OCT has been researched and developed which includes an adaptive optical (AO) system that measures the aberrations of an eye to be examined with a wavefront sensor in real time and corrects aberrations of measurement light or its return light occurring in the eye to be examined with a wavefront correction device and which extracts a microstructure of the retina at high resolution. For example, the AO-OCT is discussed in "Ultrahigh-resolution optical coherence tomography with monochromatic and chromatic aberration correction", Opt. Express 16, 8126 (2008).

Furthermore, in a Fourier domain OCT apparatus discussed in "Adaptive optics optical coherence tomography for high-resolution and high-speed 3D retinal in vivo imaging", R. Zawadzki et al., Optics Express, Vol. 13, PP. 8532-8546, an adaptive optical system and a chromatic aberration correction lens are used to satisfy both of a transverse resolution and a longitudinal resolution (axial resolution).

Since the optical system of the human eyeball (the cornea, the crystalline lens, and the vitreous body) is inhomogeneous and, when an image of the fundus is captured, aberrations occur in the light wave front of light with which to capture an image of the fundus, it is usually difficult to capture an image of the retina of the fundus at high resolution.

Aberrations occurring in the wave-front of light passing through the eyeball depend on the angle of incidence to the pupil, and the angle deemed to cause almost the constant aberrations relative to the angle of incidence is said to be within several degrees (equivalent to 1 mm or less in the length on the retina). Since it is difficult to switch aberration correction, which is performed by the wavefront correction device, at high speed according to the angle of incidence of capturing light to the pupil position, the capturing angle of view of the OCT using an AO is limited to several degrees or less, so that the capturing angle of view is limited to a narrower range as compared with the angle of view of a usual OCT (10 mm or more). Moreover, since the angle of view is narrow and it is impossible to concurrently capture an image of characteristic structures (for example, the bifurcation of a blood vessel and the optic disc), it may be in some cases difficult to identify the image capturing position of the fundus only based on high-resolution tomographic images captured by using an AO.

To solve the latter issue, there has been proposed an OCT apparatus which acquires a tomographic image in which a region high in transverse resolution relative to the depth direction is not limited, by combining a plurality of tomographic images acquired at respective different focus positions. Japanese Patent No. 5,743,411 discusses an adaptive optical OCT which acquires a plurality of tomographic images at respective different focus positions by moving a focusing lens and combines the plurality of tomographic images.

Furthermore, in AO-OCT, it is necessary to increase the numerical aperture (NA) to a value larger than that of a usual OCT, so that the depth of focus becomes shallow. Therefore, it is necessary to set the steps of focus positions finer than that in a conventional OCT (discussed in, for example, Japanese Patent Application Laid-Open No. 2010-169503 and Japanese Patent Application Laid-Open No. 2012-213449) which combines a plurality of striped images captured at respective different focus positions.

Since such tomographic images high in transverse resolution require high-density sampling, the image capturing range in the transverse direction naturally becomes narrow. In "The Cellular Origins of the Outer Retinal Bands in Optical Coherence Tomography Images", No. 12, Vol. 55, December, 2014, the image capturing range in the transverse direction is 210 µm, and, in "Adaptive optics optical coherence tomography for in vivo mouse retinal imaging", Journal of biomedical optics 18, No. 5 (2013), the image capturing range in the transverse direction is about in the range of 200 µm to 300 µm. In "Ultrahigh-resolution optical coherence tomography with monochromatic and chromatic aberration correction", Opt. Express 16, 8126 (2008), the image capturing range in the transverse direction corresponds to an angle of view of up to 4° (equivalent to about 1,200 µm), but, since the resolution is 500 lines/frame, in order to acquire an image with a resolution of several µm, it may be necessary to further narrow the image capturing range. With respect to such a tomographic image having a narrow image capturing range, a need to explicitly indicate an image capturing position (image capturing range) on the retina arises, so that a method of displaying, with a frame border, the position of a tomographic image with a high transverse resolution on a separately captured wide-angle tomographic image or a method of displaying images in a superimposed manner is used.

In the case of AO-OCT, since, as mentioned above, the angle of view is narrow and the depth of focus is shallow, examining the vessel structure of a wide range including the retinal surface and the depth direction or the form distribution of photoreceptor cells may be time-consuming and troublesome, and the burden on the patient may become large. Moreover, it may be difficult to identify the image capturing position of a captured high-resolution tomographic image.

Accordingly, an image capturing apparatus capable of efficiently performing image capturing to visualize a vessel structure of the retina and to visualize an anatomical structure or lesion is expected.

Furthermore, while a method of capturing a plurality of tomographic images at respective different focus positions requires a long image capturing time, a tomographic image with a high transverse resolution relative to a wide depth region is not necessarily needed for medical examination or diagnosis. For example, in the case of image capturing specialized for observation of photoreceptor cells, the transverse resolution only needs to be high in a depth region of several tens of µm from the cone outer segment tip (COST) line (a line corresponding to the cone cell outer segment tip) to the photoreceptor inner segment/outer segment (IS/OS) line (a line corresponding to the photoreceptor inner segment/outer segment junction line), and this is sustainable with image capturing at one focus position.

However, in a tomographic imaging system using a shallow depth of focus, the transverse resolution abruptly deteriorates as the image becomes out of focus as compared with a usual tomographic imaging system using a deep depth of focus. Accordingly, with respect to tomographic images with a shallow depth of focus, unless, for example, combining of a plurality of images captured at respective different focus points is performed, unnecessary regions with a remarkably low transverse resolution may be displayed to the user.

### SUMMARY OF THE INVENTION

Aspects of the present invention are generally directed to providing a tomographic image acquisition apparatus capable of easily designating the acquisition position of a high-resolution tomographic image using a low-resolution tomographic image.

Another aspect of the present invention is directed to providing a tomographic image acquisition apparatus capable of displaying the acquisition position of a high-resolution tomographic image in an easily understandable manner.

A further aspect of the present invention is directed to providing a tomographic image acquisition apparatus enabling, when observing a high-resolution tomographic image captured via an optical system having a high NA and a shallow depth of focus, both checking a required region with a high-resolution tomographic image and checking the position of a high-resolution tomographic image in the fundus.

According to a first aspect of the present invention, there is provided a tomographic image acquisition apparatus as specified in claims 1 to 11 and 14 to 24. According to a second aspect of the present invention, there is provided a tomographic image acquisition method as specified in claims 12, 13, 25, and 26.

Further features of the present invention will become apparent from the following description of embodiments with reference to the attached drawings.

### BRIEF DESCRIPTION OF THE DRAWINGS

Fig. 1 is a schematic configuration diagram of an ophthalmic imaging apparatus according to first and second embodiments of the present invention.
Fig. 2 is a configuration diagram of an optical system in the first and second embodiments of the present invention.
Fig. 3 is a flowchart illustrating processing for generating an optical coherence tomography (OCT) image.
Fig. 4 is a schematic explanatory diagram of a display form provided by a display control unit in the first and second embodiments of the present invention.
Fig. 5 is a flowchart illustrating a shooting operation in the first and second embodiments of the present invention.
Fig. 6 is a diagram illustrating an example of segmentation of a tomographic image.
Figs. 7A, 7B, and 7C are diagrams illustrating a shooting sequence and the number of shots in the first and second embodiments of the present invention.
Fig. 8 is a schematic view illustrating a functional configuration of an image generating apparatus according to a third embodiment of the present invention.
Fig. 9 is a schematic view illustrating a hardware configuration of the image generating apparatus according to the third embodiment of the present invention.
Fig. 10 is a flowchart illustrating an image compositing processing procedure performed by the image generating apparatus according to the third embodiment of the present invention.
Fig. 11 is a diagram illustrating a main window in a program for executing OCT image selection in the third embodiment of the present invention.
Fig. 12 is a flowchart illustrating a processing procedure for high-resolution region extraction in the third embodiment of the present invention.
Fig. 13 is a schematic view illustrating fitting processing for determining a high-resolution region in the third embodiment of the present invention.
Figs. 14A and 14B are schematic views illustrating layouts of composite images generated by the image generating apparatus according to the third embodiment of the present invention.
Fig. 15 is a schematic view illustrating another layout of composite images generated by the image generating apparatus according to the third embodiment of the present invention.
Fig. 16 is a schematic view illustrating a functional configuration of an image generating apparatus according to a fourth embodiment of the present invention.

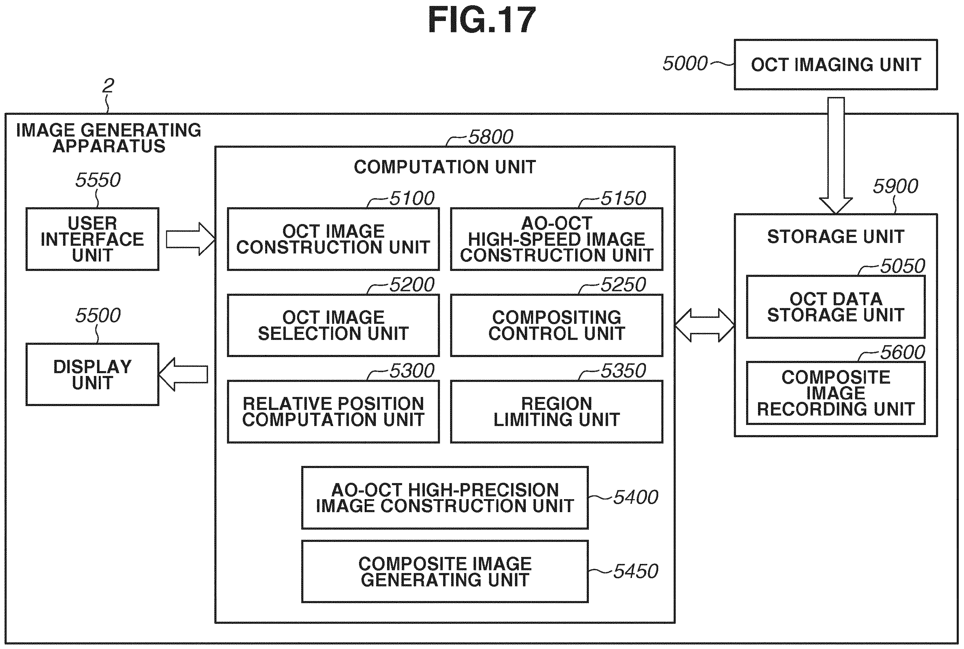

Fig. 17 is a schematic view illustrating a hardware configuration of the image generating apparatus according to the fourth embodiment of the present invention.
Fig. 18 is a schematic view illustrating a configuration of an OCT imaging unit in the fourth embodiment of the present invention.
Fig. 19 is a flowchart illustrating an image compositing processing procedure performed by the image generating apparatus according to the fourth embodiment of the present invention.
Fig. 20 is a schematic view illustrating a relationship of parameters for determining a high-resolution region in the fourth embodiment of the present invention.

### DESCRIPTION OF THE EMBODIMENTS

Various embodiments, features, and aspects of the invention will be described in detail below with reference to the drawings. However, the present invention is not limited to these embodiments.

### [Overall Configuration of Ophthalmic Imaging Apparatus]

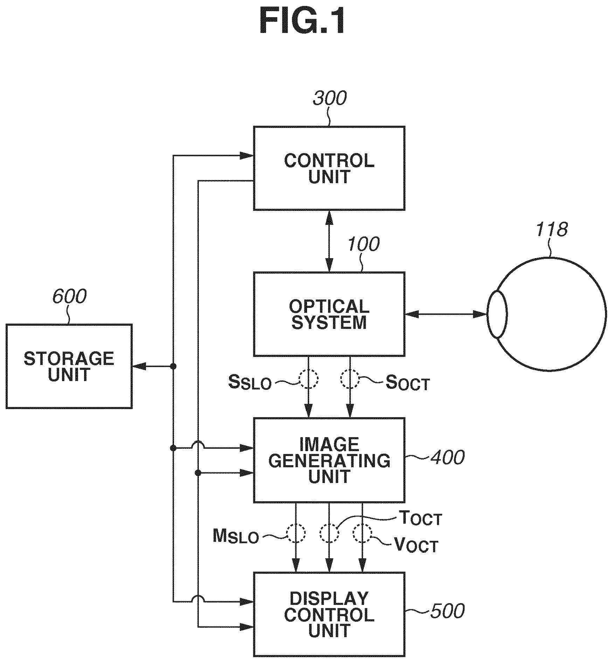

Fig. 1 is a schematic configuration diagram of an ophthalmic imaging apparatus serving as a tomographic image acquisition apparatus according to a first embodiment of the present invention.

Referring to Fig. 1, the ophthalmic imaging apparatus includes an optical system 100, a control unit 300, an image generating unit 400, and a display control unit 500. The optical system 100, which is controlled by the control unit 300, irradiates an eye to be examined 118 with measurement light and detects return light from the eye to be examined 118. The image generating unit 400 receives an electrical signal corresponding to the return light from the optical system 100, performs image processing on the electrical signal to generate a tomographic image T_{OCT}, and outputs the tomographic image T_{OCT} to the display control unit 500. The display control unit 500, which includes a display device such as a liquid crystal display, displays the input image. Furthermore, the generated image is stored in a storage unit 600 together with information identifying the eye to be examined 118.

The ophthalmic imaging apparatus illustrated in Fig. 1 can be implemented by a personal computer (PC) connected to hardware having a specific function. For example, the optical system 100 can be implemented by hardware, and the control unit 300, the image generating unit 400, and the display control unit 500 can be implemented by a software module installable in a PC connected to the hardware. While, in the following embodiments, functions are implemented by a central processing unit (CPU) of the PC executing the software module, the present invention is not limited to such a method. The image generating unit 400 can be implemented by dedicated hardware, such as an application specific integrated circuit (ASIC), and the display control unit 500 can be implemented by a dedicated processor, such as a graphics processing unit (GPU), different from the CPU. Furthermore, the connection between the optical system 100 and the PC can be configured and implemented via a network without changing the gist of the present invention.

Next, a detailed configuration of each unit is described.

### <Configuration of Optical System 100>

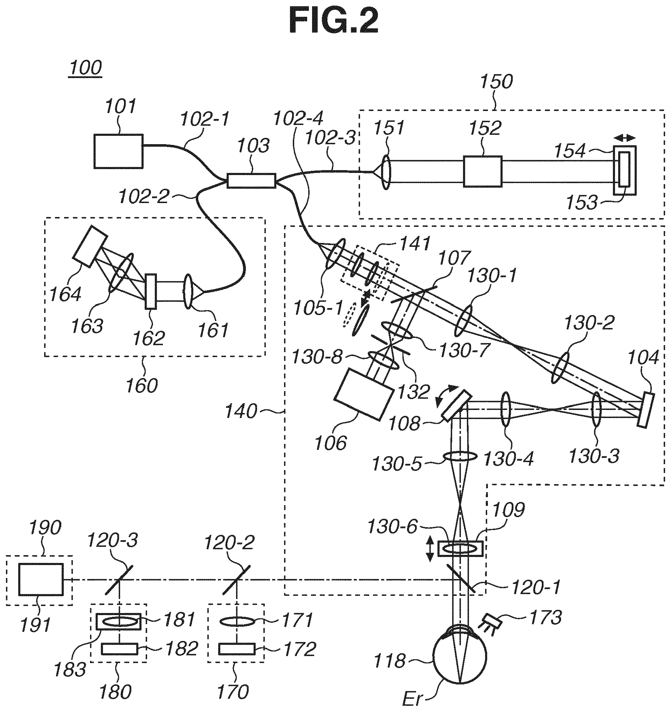

A configuration of the optical system 100 is described as follows with reference to Fig. 2. Principal constituent elements of the optical system 100 include a light source 101, an adaptive optics (AO) and measurement unit 140, a reference optical system 150, a light-receiving optical system 160, an anterior eye portion observation unit 170, a fixation target optical system 180, and a scanning laser ophthalmoscope (SLO) portion 190. Furthermore, unless otherwise described, each unit operates under the control of the control unit 300.

The light source 101 is a low-coherence light source, for which, for example, a super luminescent diode (SLD) light source with a center wavelength of 840 nm and a wavelength width of 100 nm is used. While, in the first embodiment, the SLD light source is used, instead, for example, a titanium-sapphire laser can be used.

Light emitted from the light source 101 is guided to a beam splitter 103 via a single mode fiber (SM fiber) 102-1 and is then branched into measurement light and reference light. In the first embodiment, the beam splitter 103 is configured with a fiber coupler. The branching ratio of the beam splitter 103 is 90 (reference light) : 10 (measurement light).

The branched measurement light is guided to the AO and measurement unit 140 via an SM fiber 102-4 and is then caused by a collimator 105-1 to become parallel light, which is then made incident on a beam diameter varying unit 141.

The beam diameter varying unit 141 is a unit which changes the beam diameter of measurement light to vary the NA of measurement light with which to irradiate the fundus Er of the eye to be examined 118. In the first embodiment, the beam diameter varying unit 141 is configured with a plurality of lenses located at adjustable positions in a detachable manner. The beam diameter varying unit 141 is configured to be able to communicate with the control unit 300 and is thus able to change the beam diameter of measurement light in response to an instruction from the control unit 300. This enables switching between an OCT mode, which performs image capturing at a transverse resolution of about the same level of that of an ordinary OCT, and an AO-OCT mode, which performs image capturing at a transverse resolution higher than that of the transverse resolution of the OCT mode. In the OCT mode, image capturing is performed with the beam diameter of measurement light made narrow and the NA made low, and, in the AO-OCT mode, image capturing is performed with the beam diameter of measurement light made wide and the NA made high. It is desirable that the beam diameter varying unit 141 be operated in such a manner as to set the beam diameter in the OCT mode to 2 mm or less and the beam diameter in the AO-OCT mode to 6 mm or more on the pupil of the eye to be examined 118. However, to cope with a case where it is impossible to widen the pupil of the eye to be examined 118 up to 6 mm or more or the entrance pupil of the eye to be examined 118 is narrow, for example, due to a disease such as cataract, a shooting mode which performs AO-OCT with a beam diameter of 2 mm to 6 mm can also be provided. Furthermore, in the first embodiment, the beam diameter in the OCT mode is assumed to be 1 mm (a spot diameter of 20 µm on the fundus), and the beam diameter in the AO-OCT mode is assumed to be 6 mm (a spot diameter of 3.5 µm on the fundus).

Next, the measurement light passes through a light splitting unit 107 and is then guided to a wavefront correction device 104, a scan optical system 108, and the eye to be examined 118 via relay optical elements 130-1 to 130-6. In the first embodiment, each of the relay optical elements 130-1 to 130-6 is configured with a lens. The relay optical elements 130-1 to 130-6 are adjusted in such a manner that the entrance pupils of the wavefront correction device 104, the scan optical system 108, and the eye to be examined 118 are approximately conjugate in phase with each other.

The scan optical system 108 is assumed to be able to perform two-dimensional, x-y, scan and can be a scanner which performs two-dimensional scan with a single mirror or can be configured with a plurality of scanners. In the first embodiment, the scan optical system 108 is configured with a galvano scanner in each of the y (vertical) direction and the x (horizontal) direction. Furthermore, the scan optical system 108 is also able to change (steer) the shooting position based on an instruction from the control unit 300.

The relay optical elements 130-5 and 130-6 have a function to adjust the focus position of measurement light on the fundus Er. The relay optical element 130-6 is fixed onto a stage 109, and the focus position can be adjusted by moving the stage 109 along the optical axis direction. Furthermore, while, in the first embodiment, the focus position is adjusted by moving the lens, the lens can be fixed and a Badal optometer having a mechanism which adjusts an optical path length can also be used.

Furthermore, in order to prevent reflected light on the lens surface from becoming stray light, the relay optical elements 130-1 to 130-6 can be a configuration using, for example, mirrors.

The scan optical system 108 and the stage 109, which are controlled by the control unit 300, are able to perform scanning within an intended range of the fundus Er of the eye to be examined 118 with measurement light. The measurement light is made incident on the eye to be examined 118 and is focused at an intended depth position of the fundus Er by the relay optical element (focus lens) 130-6 mounted on the stage 109.

The measurement light with which the fundus Er has been irradiated is reflected and scattered to become return light, and the return light tacks back the relay optical elements 130-1 to 130-6 in reverse order and then returns to the light splitting unit 107. Part of the return light split by the light splitting unit 107 is guided to a wavefront sensor 106 via relay optical elements 130-7 and 130-8 and an aperture 132. The relay optical elements 130-7 and 130-8 are adjusted in such a manner as to be approximately conjugate in phase with the entrance pupils of the wavefront correction device 104, the scan optical system 108, and the eye to be examined 118. The aperture 132 is inserted so as to prevent stray light from entering the wavefront sensor 106. Furthermore, the branching ratio of the light splitting unit 107 is determined by the amount of light incident on the eye to be examined 118, the use efficiency (throughput) of light by the AO and measurement unit 140, the sensitivity of the wavefront sensor 106, and the throughput of the light-receiving optical system 160 and is 90 (transmitted) : 10 (reflected) in the first embodiment.

The return light deriving from the measurement light and passing through the light splitting unit 107 is made incident on the beam splitter 103 via the beam diameter varying unit 141, the collimator 105-1, and the SM fiber 102-4.

On the other hand, the reference light split by the beam splitter 103 is caused to exit via an SM fiber 102-3 and is then caused to become parallel light by a collimator 151. The reference light is reflected by a mirror 153 via a dispersion-compensating glass 152. The mirror 153 is located on a stage 154, and the stage 154 is controlled by the control unit 300 in such a manner as to be driven in the optical axis direction according to, for example, differences in eye axial length of subjects to adjust the coherence gate position (a difference in optical path length between measurement light and reference light). Furthermore, while, in the first embodiment, the optical path length of reference light is changed, an optical path length difference between the optical path of measurement light and the optical path of reference light only needs to be changed.

The reference light reflected from the mirror 153 tracks back the optical path in reverse order and is made incident on the beam splitter 103 via the dispersion-compensating glass 152, the collimator 151, and the SM fiber 102-3. In the beam splitter 103, the return light deriving from the measurement light and the reference light are combined to become interference light, which then enters the light-receiving optical system 160.

The interference light entering the light-receiving optical system 160 is made incident on a detector 164 via a collimator 161, a grating 162, and an imaging lens 163. The interference light is separated into spectrums by the grating 162, is received by the detector 164 for each spectral component of the interference light, and is then converted into a signal S_{OCT}, which is input to the image generating unit 400.

### <AO Operation>

An operation of the AO system in the AO-OCT mode is described as follows.

In the first embodiment, a Shack-Hartmann sensor is used as the wavefront sensor 106. In the Shack-Hartmann sensor, incident light (in the first embodiment, light split from return light arising from measurement light) is split by a microlens array, and the respective split pieces of measurement light are focused on a two-dimensional sensor. An output from the two-dimensional sensor is visualized as an image in which light-focused points are arranged side by side, which is called a "Hartmann image". The wavefront sensor 106 is connected to the control unit 300, and the control unit 300 reads the Hartmann image. The control unit 300 obtains aberrations of return light arising from measurement light (aberrations occurring in the eye to be examined) based on the amount of movement of each light-focused point from the associated reference point.

The control unit 300 calculates an input signal for the wavefront correction device 104 (a drive signal for canceling aberrations) based on the obtained aberrations, and drives the wavefront correction device 104 in such a manner as to reduce the aberrations. A series of operations including acquisition of the Hartmann image by the wavefront sensor 106, calculation of aberrations by the control unit 300, and driving of the wavefront correction device 104 is repeatedly performed and is constantly continued in such a manner that aberrations of return light arising from measurement light are reduced. Furthermore, the input signal for the wavefront correction device 104 calculated by the control unit 300 can be a signal including a superposed signal for transforming the wave front of return light arising from measurement light into an intended shape, such as a concave surface shape (defocus) for changing focusing on the fundus Er.

### <Anterior Eye Portion Observation>

The eye to be examined 118 is irradiated with illumination light emitted from an anterior eye portion illumination light source 173, and an image of the anterior eye portion of the eye to be examined 118 is captured by the anterior eye portion observation unit 170. In the first embodiment, a light-emitting diode (LED) with a center wavelength of 740 nm is used as the anterior eye portion illumination light source 173. Illumination light from the anterior eye portion illumination light source 173 is successively reflected by dichroic mirrors 120-1 and 120-2 and is then made incident on the anterior eye portion observation unit 170. The anterior eye portion observation unit 170 is configured with an imaging lens 171 and a two-dimensional capturing device 172, and is adjusted to capture an image of the anterior eye portion of the eye to be examined 118. A signal output from the two-dimensional capturing device 172 is input to the display control unit 500 via the control unit 300 and is thus displayed on the display device. A split prism can be introduced into the anterior eye portion observation unit 170 so as to facilitate position adjustment of the eye to be examined 118 in a direction along the optical axis thereof.

Before starting OCT imaging, the user turns on the anterior eye portion illumination light source 173 and performs position adjustment of the eye to be examined 118 using the obtained image of the anterior eye portion. Furthermore, the user can suspend OCT imaging as appropriate and can turn on the anterior eye portion illumination light source 173 to confirm the position of the eye to be examined 118. Upon completion of the position adjustment, the user turns off the anterior eye portion illumination light source 173 and completes photographing performed by the anterior eye portion observation unit 170.

### <Fixation Target Optical System>

The fixation target optical system 180 includes an optical element 181 and a light-emitting type organic electroluminescence (EL) display module 182. The display module 182 to be used includes, for example, a liquid crystal display and an LED array.

A pattern displayed in a lighting manner on the display module 182 according to an instruction from the control unit 300 is projected onto the fundus Er at an appropriate magnification via the optical element 181 and dichroic mirrors 120-3, 120-2, and 120-1. Changing the lighting position of the pattern enables guiding the visual fixation of the eye to be examined 118 and performing adjustment in such a way as to be able to capture an intended position of the fundus Er. The pattern to be used includes, for example, a cross-like figure, a circular form, and a quadrilateral, and can be desirably a pattern most easily perceivable by the eye to be examined 118. Furthermore, adjustment can be performed to facilitate perception by the eye to be examined 118 by, for example, changing the size of a pattern or inserting a color filter.

The optical element 181 is able to adjust focusing on the fundus Er according to an instruction from the control unit 300. The optical element 181 is mounted on a stage 183, and the stage 183 is moved in conjunction with the stage 109 of the AO and measurement unit 140.

### <SLO Portion>

The SLO portion 190 captures an image of a wide range (with an angle of view of about 40 degrees × 40 degrees) of the fundus Er. An SLO unit 191 is configured with, for example, a light source, a two-dimensional scanner, and a detector, and acquires about fifteen SLO images per one second. An SLO signal S_{SLO} acquired by the SLO unit 191 is converted by the image generating unit 400 into an SLO image M_{SLO}, which is then input to the display control unit 500 and is thus displayed on the display device included in the display control unit 500.

The acquired SLO image can be used to confirm the shooting position of, for example, a disease site or to designate the shooting position with an input device, such as a keyboard or mouse (not illustrated).

### <Tracking>

The optical system 100 performs tracking, which detects any deviation of the shooting position due to, for example, involuntary eye movement of the eye to be examined 118 and adjusts the shooting position in real time. A procedure of the tracking is described as follows.

One of SLO images of the fundus Er acquired by the SLO unit 191 is selected as a reference image, which is then stored in the storage unit 600. The reference image is desirably an image with a high signal-to-noise ratio (S/N ratio) and a little image distortion, and an average image of a plurality of SLO images can be used as the reference image.

When shooting by the SLO unit 191 is started, the acquired SLO image is sent to the control unit 300, in which the amount of positional deviation is obtained based on a calculation result of a correlation value with respect to the reference image. The calculation of the correlation value can be performed while relatively shifting pixels between the reference image and the SLO image or can be performed using a phase only correlation (POC) method using Fourier transform. Furthermore, since high-speed calculation is required, a Field Programmable Gate Array (FPGA) unit can be used or a graphics processing unit (GPU) can be used.

The control unit 300 sends an instruction for canceling the positional deviation to the scan optical system 108 based on the amount of positional deviation obtained by calculation, so that the galvano scanner for the x direction and the galvano scanner for the y direction are driven to adjust the shooting position on the fundus Er.

Since tracking is performed based on an SLO image in which a wide region of the fundus Er is captured, even if the shooting position deviates to a great extent, the amount of positional deviation can be obtained, so that the same position can be stably captured without deviation from a frame.

### <Control Unit 300>

Next, the control unit 300 is described. As mentioned above, in the first embodiment, the control unit 300 implements its functions with a CPU executing a software module, controls each unit of the optical system 100, and controls the entire operation of the ophthalmic imaging apparatus according to the first embodiment. Furthermore, the control unit 300 is assumed to receive inputs from the user operating the ophthalmic imaging apparatus. More specifically, the user inputs, for example, information such as a patient identifier (ID) for identifying an eye to be examined, parameters required for imaging, and selection of a pattern used to scan the fundus to the control unit 300 with a device such as a keyboard or mouse (not illustrated). The control unit 300 controls each unit based on the inputs from the user, and has a function to store data, such as the obtained signal or image, in the storage unit 600.

### <Image Generating Unit 400>

The image generating unit 400 performs various processing operations on signals output from the optical system 100 to generate and output images concerning an eye to be examined.

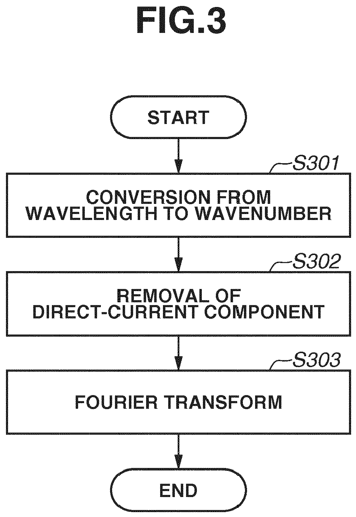

In generating an OCT image, as illustrated in Fig. 3, in step S301, the image generating unit 400 re-performs mapping to convert the signal S_{OCT} related to wavelengths into a signal related to wavenumbers, then in step S302, the image generating unit 400 removes a direct-current component from the signal, and, then in step S303, the image generating unit 400 performs Fourier transform on the signal to acquire an OCT image T_{OCT}. Furthermore, the image generating unit 400 performs, for example, position adjustment or averaging of a plurality of OCT images and processing for combining a plurality of images captured at respective different fundus positions to generate a three-dimensional OCT image V_{OCT}.

In generating an SLO image, the image generating unit 400 converts the signal S_{SLO} into two-dimensional data in synchronization with a signal output from the scanner, and performs, for example, processing for converting the data into an SLO image M_{SLO}.

### <Display Control Unit 500>

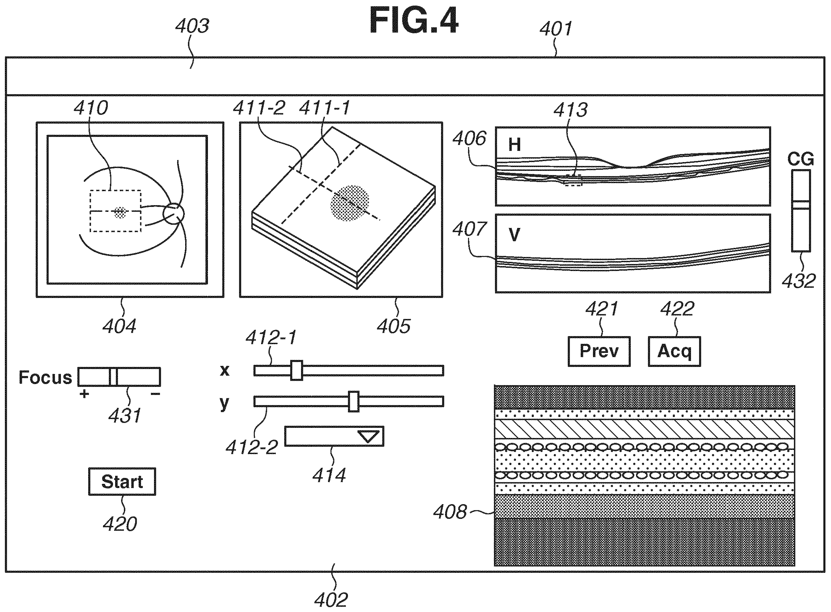

Next, the display control unit 500 is described. As mentioned above, the display control unit 500 includes a display device such as a liquid crystal display, and displays an image input from the image generating unit 400. Fig. 4 illustrates a configuration of a screen displayed by the display control unit 500. Furthermore, apart from the screen illustrated in Fig. 4, an input screen for entering information identifying an eye to be examined, such as a patient ID, to be input via the control unit 300 is required, but, since this can be configured in a known structure and is not a main aspect of the present invention, the description thereof is omitted.

Referring to Fig. 4, a screen configured with an image display region 402 and an eye-to-be-examined information display region 403 is displayed on a display device 401. Information about, for example, a patient ID, name, and age is displayed in the eye-to-be-examined information display region 403. Image display areas 404, 405, 406, 407, and 408, and buttons 420, 421, and 422, sliders 412 (412-1 and 412-2), 431, and 432, and a pull-down menu 414, which are user interfaces that are operable by the user, are arranged in the image display region 402.

The images displayed in the respective image display areas and the functions of the respective user interfaces are described in the flow of an imaging operation described below.

### [Imaging Operation]

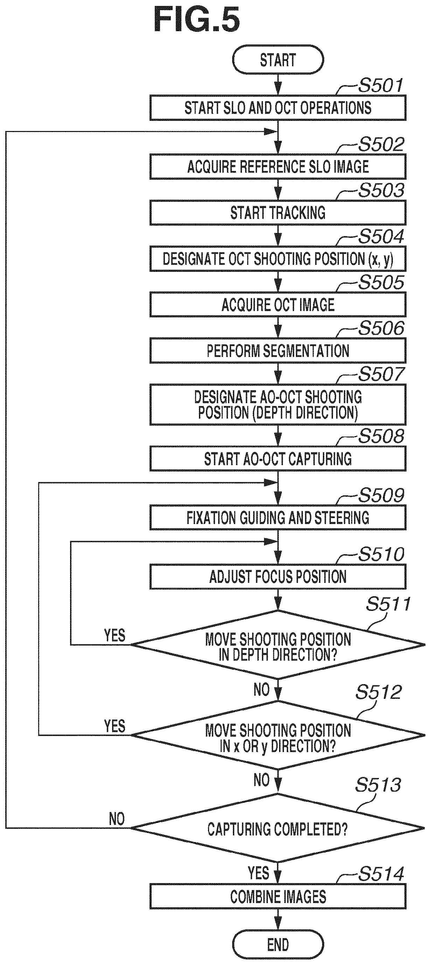

Next, an operation of the ophthalmic imaging apparatus according to the first embodiment is described with reference to a flowchart illustrated in Fig. 5.

In step S501 (Start SLO and OCT Operations), the ophthalmic imaging apparatus starts operations of, for example, the light source and the scanner unit of the SLO unit 191 and two-dimensionally irradiates the fundus Er of the eye to be examined 118 with light. A preview of the SLO image is displayed in the image display area 404, so that the user can confirm the shooting position and image quality of the fundus Er.

Moreover, the ophthalmic imaging apparatus starts operations of the light source 101 and the scan optical system 108. Furthermore, the ophthalmic imaging apparatus performs initialization in such a manner that a reflecting surface of the wavefront correction device 104 becomes flat. A preview of the OCT image is displayed in the image display area 405.

In step S502 (Acquire Reference SLO Image), the ophthalmic imaging apparatus adjusts the SLO unit 191 and the fixation target optical system 180 in such a manner that an intended shooting position in the fundus Er is acquired. The ophthalmic imaging apparatus fixes the lighting position of the fixation target optical system 180 and photographs the intended shooting position with the steering operation of the SLO unit 191.

Next, the ophthalmic imaging apparatus makes an adjustment to the focus position for SLO. The adjustment to the focus position can be made automatically or manually, and the user can select the automatic or manual adjustment. In the case of the automatic adjustment to the focus position, the control unit 300 performs driving to move the SLO focus position to a focus position in which the root mean square (RMS) value of the SLO image becomes maximum.

The ophthalmic imaging apparatus sets the SLO image acquired after completion of the focus adjustment as a reference SLO image, and stores the reference SLO image together with the RMS value in the storage unit 600. Moreover, in the case of the manual adjustment to the focus position, the control unit 300 performs driving in such a way as to move the focus position for SLO based on a value directed by the user via the slider 431. The ophthalmic imaging apparatus sets the SLO image acquired after it is determined that there is no input from the user within a previously set time as a reference SLO image, and stores the reference SLO image together with the RMS value in the storage unit 600. Furthermore, in a case where a reference SLO image is already stored in the storage unit 600, the ophthalmic imaging apparatus compares the newly-acquired RMS value with the stored RMS value, and stores the reference SLO image having a higher RMS value in the storage unit 600 together with that RMS value.

Since focusing for OCT is interlocked with focusing for SLO, the control unit 300 performs driving to move the stage 109 according to adjustment of the focus position for SLO.

In step S503 (Start Tracking), the ophthalmic imaging apparatus starts tracking with the reference SLO image acquired in step S502 set as a reference image.

In step S504 (Specify OCT Shooting Position (x, y)), since a rectangular mark 410 indicating the shooting position for OCT is displayed in the image display area 404 in a superimposed manner, the user designates the shooting position (a rectangular area) by moving the mark 410 with an input device such as a mouse. The control unit 300 drives the scan optical system 108 based on the position of the mark 410, and performs prescan within the shooting range designated by the mark 410. The prescan is an operation performed while reducing the number of OCT images acquired by a single two-dimensional scan, and, in the first embodiment, the control unit 300 acquires an OCT image at the middle of the shooting range and previews the acquired OCT image in the image display area 405. Moreover, the control unit 300 drives the stage 154 based on the luminance value of the OCT image to adjust the coherence gate in such a manner that a tomogram of the retina of the fundus Er falls within the image.

Furthermore, while, in the first embodiment, a single OCT image is previewed, a plurality of OCT images can be configured to be previewed. For example, displaying OCT images of the outer peripheral portion or central cross portion of the shooting range designated with the mark 410 enables determining whether focusing or coherence gate adjustment is appropriate. Furthermore, the user can operate the slider 432 to manually fine-tune the coherence gate.

In step S505 (Acquire OCT Image), in response to the user pressing the button 420, the ophthalmic imaging apparatus starts acquiring three-dimensional OCT data. The ophthalmic imaging apparatus acquires the data about the shooting range designated by the mark 410 based on predetermined parameters, such as the pixel resolution, acquiring speed, and number of times of acquisition.

In step S506 (Perform Segmentation), the data acquired in step S505 is sent to the image generating unit 400 and is then visualized as an image, which is thus displayed in the image display area 405. Moreover, segmentation (site information acquisition) is performed, and superimposition of segmentation lines can be switched between being displayed and being hidden.

Here, segmentation about the retina is specifically described.

The image generating unit 400 individually applies a median filter and a Sobel filter to a tomographic image extracted from the OCT image and targeted for processing to generate respective images (hereinafter respectively referred to as a "median image" and a "Sobel image"). Next, the image generating unit 400 generates profiles for each A-scan from the generated median image and Sobel image. The profiles include a profile of luminance values in the median image and a profile of gradients in the Sobel image. Then, the image generating unit 400 detects a peak in the profile generated from the Sobel image. The image generating unit 400 detects a boundary of each region of the retina layer by referring to a profile of the median image corresponding to portions before and after the detected peak or between the detected peaks.

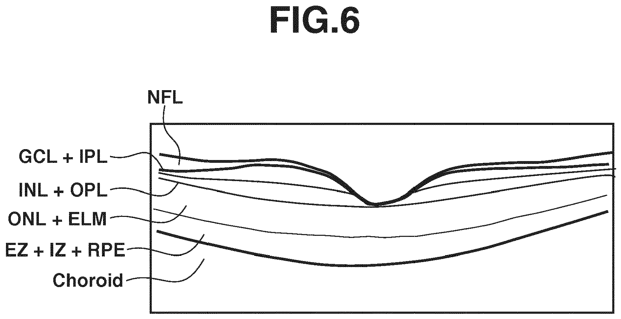

A result of segmentation is described with reference to Fig. 6. Fig. 6 illustrates a tomographic image averaged in luminance, in which segmentation lines are superimposed with solid lines. In the first embodiment, segmentation results in detection of six layers. The particulars of six layers include (1) a nerve fiber layer (NFL), (2) a combined layer of a ganglion cell layer (GCL) and an inner plexiform layer (IPL), (3) a combined layer of an inner nuclear layer (INL) and an outer plexiform layer (OPL), (4) a combined layer of an outer nuclear layer (ONL) and an external limiting membrane (ELM), (5) a combined layer of an ellipsoid zone (EZ), an interdigitation zone (IZ), and a retinal pigment epithelium (RPE), and (6) a choroid.

Furthermore, the segmentation described in the first embodiment is merely an example, and another method, such as segmentation using a Dijkstra's algorithm, can be used. Furthermore, the number of layers to be detected can be optionally selected.

In step S507 (Designate AO-OCT Shooting Position), dashed lines 411-1 and 411-2 superimposed on the image in the display area 405 are drawn in such a way as to extend along x and y directions of the three-dimensional OCT image, and the respective positions thereof can be adjusted by operating the sliders 412-1 and 412-2. When values directed with the sliders 412 are updated, tomographic images in the respective positions corresponding to the dashed lines 411 are automatically drawn in the display area 406 (horizontal direction H, which corresponds to the dashed line 411-2) and the display area 407 (vertical direction V, which corresponds to the dashed line 411-1). The user makes adjustments using the sliders 412 in such a manner that the shooting position for AO-OCT falls within the tomographic images. Furthermore, the user can operate the pull-down menu 414 to select a single one or a combined plurality of layers obtained by segmentation and to highlight displaying of an intended layer of the retina by, for example, making a non-selected layer transparent or translucent, so that the adjustment of the shooting position can be facilitated.

Furthermore, to enable easily designating the shooting position for AO-OCT, a range for drawing a tomographic image can be narrowed to an extent of about 1 mm from the position at which the dashed lines 411-1 and 411-2 intersect with each other. Moreover, to enable further easily designating the position of a tomographic image, the dashed lines 411-1 and 411-2 can be projected along the curvature of the retina. Additionally, the dashed lines 411-1 and 411-2 can be configured to enable designating an optional direction with an input device such as a mouse, or, to enable easily designating, for example, a disease site, a tomographic image generated from a three-dimensional OCT image using, for example, interpolation can be displayed in the display area.

Designating the shooting position for AO-OCT is performed by the user moving a rectangular mark 413 displayed in a superimposed manner in the display area 406 or display area 407. The mark 413 is displayed in any one of the display areas 406 and 407 which the mouse cursor operated by the user enters, according to the position at which the dashed lines 411-1 and 411-2 intersect with each other. Furthermore, when the user presses the button 421, which is used to switch between the OCT mode and the AO-OCT mode, the ophthalmic imaging apparatus transitions to the AO-OCT mode.

When the ophthalmic imaging apparatus transitions to the AO-OCT mode, the control unit 300 drives the beam diameter varying unit 141 to change the beam diameter and starts an operation for AO. The control unit 300 performs preview capturing for the AO-OCT mode based on parameters, such as a previously set scan amplitude and scan speed. In the first embodiment, the ophthalmic imaging apparatus captures the shooting position at the center of the region designated by the mark 413 and previews an AO-OCT image in the display area 408. Herein, generation of the AO-OCT image is described. As with the case of an ordinary OCT image, the ophthalmic imaging apparatus obtains a tomographic image for A-scan by performing fast Fourier transform (FFT) on a signal corresponding to interference light. The ophthalmic imaging apparatus sets the depth position in an OCT image based on the set focus position. The ophthalmic imaging apparatus extracts, as a high-resolution tomographic image, an OCT image having a width (a width in the image height direction) corresponding to the width of a longitudinal high-resolution region in the retina depth direction of a tomographic image centering on the position corresponding to the set depth position in the tomographic image for A-scan. The ophthalmic imaging apparatus repeats this processing for A-scans corresponding to the breadth of the image to generate an AO-OCT image. Furthermore, further details thereof are described below in a third embodiment.

The ophthalmic imaging apparatus calculates the lighting position of the fixation target optical system 180 and the movement distance of the shooting position caused by steering (hereinafter referred to as a "steering amount") based on the shooting range designated by the mark 413, and the control unit 300 automatically makes an adjustment to the shooting position. Furthermore, in the first embodiment, the lighting position of the fixation target optical system 180 is set according to an intersection point of the dashed lines 411-1 and 411-2. Moreover, the focus position during AO-OCT imaging is set based on the coherence gate position during OCT capturing (the upper end of an image) and a high reflecting layer of the retina (at least one of, for example, stratum opticum, IS/OS, and RPE) and is set to a position in which the position of the mark 413 is obtained from a relative position in a space between the two portions. Additionally, the ophthalmic imaging apparatus can adjust the focus position by superimposing previously-set defocus on the wavefront correction device 104 with the high reflecting layer used as a reference. To set the reference, after starting capturing, the ophthalmic imaging apparatus can perform through-focus capturing (capturing to be continuously performed in the depth direction). Besides, the ophthalmic imaging apparatus can be configured to previously store a setting value of the stage 109 and a defocus value of the wavefront correction device 104 by which focus is made on the high reflecting layer in the storage unit 600 and to cause the control unit 300 to operate based on the stored values.

In a case where the automatic movement to an intended shooting position is failed, for example, when guiding for fixation is less than successful, the adjustment of the lighting position of the fixation target and the steering amount can be manually performed. In a case where the eye to be examined 118 becomes out of alignment due to, for example, the adjustment of the shooting position, a configuration for prompting the user to readjust alignment can be provided.

When storing the lighting position of the fixation target optical system 180 and the steering amount in the storage unit 600 and capturing the same position, the ophthalmic imaging apparatus causes the control unit 300 to operate based on the stored lighting position and steering amount. Furthermore, to correct a difference between the designated shooting position and the automatically adjusted shooting position, a configuration for adjusting the lighting position and the steering amount based on the manually adjusted lighting position or steering amount can be provided.

In a case where the lighting position of the fixation target optical system 180 has been changed, since the reference SLO image deviates from the reference position, the ophthalmic imaging apparatus temporarily suspends tracking.

While a range for shooting designated by the mark 413 (the size of a rectangular region) is allowed to be optionally set by the user, since the angle of view is limited in the case of capturing using AO, it is necessary to divide the region of the fundus to acquire data in the case of capturing a wide range. Therefore, with respect to the size of the mark 413 which the user is allowed to specify, respective different upper limits are provided with regard to the x direction, y direction, and depth direction. If the user tries to specify a value exceeding the upper limit, the size of the mark 413 is automatically set to the upper limit or a prompt screen for informing the user of the occurrence of an error is displayed.

In step S508 (Start AO-OCT Capturing), in response to the user pressing the button 422, the ophthalmic imaging apparatus starts AO-OCT capturing. In a case where the ophthalmic imaging apparatus has not yet transitioned to the AO-OCT mode in step S507, the control unit 300 drives the beam diameter varying unit 141 to change the beam diameter and starts an operation for AO.

First, the control unit 300 determines parameters, such as the scan amplitude and scan speed, of the scan optical system 108, the number of shots, and the shooting sequence based on the shooting range designated by the mark 413.

In a case where the shooting range exceeds the maximum width of a single AO-OCT image, the control unit 300 divides the shooting range in such a manner that AO-OCT images captured at adjacent shooting positions overlap each other. Moreover, with respect to the depth direction, in consideration of a shallow depth of focus (theoretically, about 20 µm), the control unit 300 performs division into regions each of about 50 µm to 100 µm to perform capturing. The reason why each region is set larger than the theoretical value is that a change in focus is moderate even outside the range of the depth of focus. However, to shorten the shooting time, the control unit 300 can perform division into regions each of 100 µm or more.

In a case where tracking is suspended in step S507 or in a case where capturing is performed while changing the lighting position of the fixation target optical system 180 a plurality of times, the ophthalmic imaging apparatus acquires the reference SLO image before starting capturing for AO-OCT and then starts tracking.

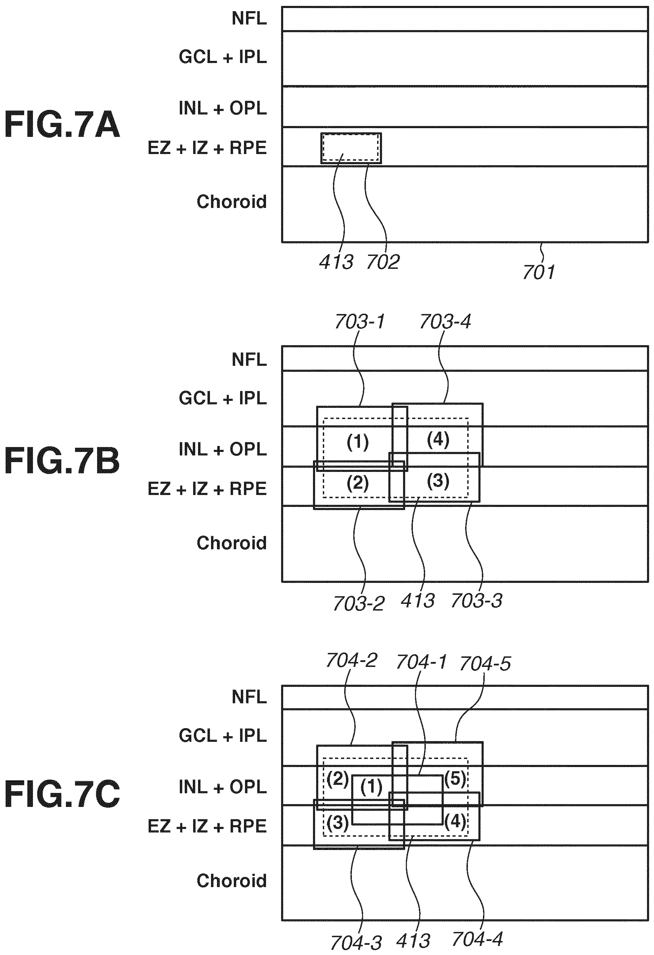

The shooting sequence in the AO-OCT mode in the first embodiment is described with reference to Figs. 7A, 7B, and 7C.

In Figs. 7A to 7C, a region (for example, a region 702) indicated by a solid line in superimposition on an OCT image 701 is a shooting range in which aberrations are deemed to be approximately constant, and is a region which is able to be captured without changing the lighting position of the fixation target optical system 180, the steering amount, and the focus position.

Fig. 7A illustrates a case where the shooting range designated by the user is narrow. The control unit 300 adjusts the fixation guiding, steering, and focus position in such a manner that the region 702 encompasses the mark 413, so that an AO-OCT image can be acquired by a single shooting operation.

Figs. 7B and 7C illustrate cases where the shooting range designated by the user is wide and a single shooting operation is not able to encompass the entire designated range. In particular, capturing is required to be performed while being moved in each of the retina in-plane direction and depth direction. In this case, to decrease the amount of change of aberrations to be corrected, the control unit 300 prioritizes adjustment of the focus position and determines the shooting sequence in such a way as to continuously perform capturing in the depth direction. Moreover, since a displacement in alignment becomes unlikely to occur, capturing can be efficiently performed.

Fig. 7B illustrates a case where capturing is performed while dividing the range designated by the mark 413 into four regions. After capturing a region 703-1, the control unit 300 moves the focus position and then captures a region 703-2. Next, the control unit 300 performs steering, then captures a region 703-3, and, finally, moves the focus position and then captures a region 703-4.

In the case illustrated in Fig. 7C, after first capturing a region 704-1, which is the center of the range designated by the mark 413, the control unit 300 captures regions 704-2 to 704-5. The region 704-1 is used as a reference image for generation of a composite image. The generation of a composite image is described below in step S514.

In step S509 (Fixation Guiding and Steering), the control unit 300 causes the fixation target optical system 180 and the scan optical system 108 to operate based on the shooting sequence determined in step S508.

In step S510 (Adjust Focus Position), the ophthalmic imaging apparatus drives the stage 109 based on an instruction from the control unit 300 and performs adjustment in such a manner that the focus position matches an intended retina layer. The ophthalmic imaging apparatus can make an adjustment to the focus position by superimposing a defocus on the wavefront correction device 104. Moreover, the ophthalmic imaging apparatus can move the coherence gate to perform adjustment in such a manner that the S/N ratio of a signal from an intended retina layer becomes high. In this instance, the ophthalmic imaging apparatus performs adjustment in such a manner that aliasing images at the coherence gate do not overlap each other at an intended shooting position. Additionally, the user is allowed to fine-tune the coherence gate position with the slider 432.

In step S511 (Move Shooting Position in Depth Direction?), the ophthalmic imaging apparatus determines whether it is necessary to perform capturing in the depth direction. If it is necessary (YES in step S511), the processing returns to step S510, in which the ophthalmic imaging apparatus re-adjusts the focus position, and if it is not necessary (NO in step S511), the processing proceeds to step S512.

In step S512 (Move Shooting Position in x or y Direction?), the ophthalmic imaging apparatus determines whether it is necessary to move the shooting position (x, y) so as to perform capturing while dividing the region or to re-perform capturing due to the failure of capturing. If it is necessary (YES in step S512), the processing returns to step S509, in which the ophthalmic imaging apparatus performs the fixation guiding and the adjustment of steering, and if it is not necessary (NO in step S512), the processing proceeds to step S513.

In step S513 (Capturing Completed?), the ophthalmic imaging apparatus determines whether capturing has been completed. If capturing has been completed (YES in step S513), the processing proceeds to step S514, and if capturing is to be continued (NO in step S513), the processing returns to step S502.

In step S514 (Combine Images), in a case where a plurality of OCT images has been acquired, the ophthalmic imaging apparatus combines regions high in contrast or S/N ratio of the respective OCT images into a composite image and displays the composite image in the display area 408.

In the case of four OCT images acquired as illustrated in Fig. 7B, the ophthalmic imaging apparatus clips and combines regions 703-1 to 703-4 to generate a composite image. The ophthalmic imaging apparatus performs compositing by position adjustment of overlapping regions of images set in step S508. Furthermore, for example, in a case where overlapping is small, the ophthalmic imaging apparatus can obtain rough combining positions by setting a low-resolution OCT image 701 as a reference image and performing position adjustment with images obtained by lowering the resolution of AO-OCT images. The ophthalmic imaging apparatus performs trimming on the composite image in such a way as to approximately match the shooting range designated by the mark 413, and displays the trimmed composite image in the display area 408.

With respect to OCT images acquired as illustrated in Fig. 7C, the ophthalmic imaging apparatus combines regions 704-2 to 704-5 using a region 704-1 as a reference image. Since the reference image is acquired at approximately the central position of the shooting range, overlapping regions become large, so that the precision of compositing can be improved.

As described above, according to the first embodiment, since it is possible to designate the acquisition position of a high-resolution tomographic image using a low-resolution tomographic image, a vessel structure or a lesion can be efficiently extracted. Furthermore, since it is possible to easily designate the position of a high-resolution tomographic image, a burden to be borne by patients can be reduced.

In the above-described first embodiment, a procedure in the case of capturing a high-resolution tomographic image has been described. In a second embodiment, processing in the case of presenting, to the user, the shooting position of a high-resolution tomographic image acquired by an ophthalmic imaging apparatus serving as a tomographic image acquisition apparatus is described.

First, a plurality of high-resolution tomographic images acquired as described in the first embodiment is stored in the storage unit 600 together with positional information indicating the shooting positions.

In response to an instruction from the user, the control unit 300 displays a list of a plurality of high-resolution tomographic images read from the storage unit 600 on the display device. Then, the control unit 300 displays a selected high-resolution tomographic image in the display area 408 illustrated in Fig. 4. Besides, the control unit 300 reads out low-resolution tomographic images and positional information stored in the storage unit 600 in association with the selected high-resolution tomographic image, and displays the low-resolution tomographic images in the respective display areas 406 and 407 based on the positional information. Then, the control unit 300 displays a rectangular mark 413, which indicates the corresponding position of the high-resolution tomographic image, on the low-resolution tomographic image displayed in the display area 406 or 407. Moreover, the control unit 300 displays dashed lines 411-1 and 411-2, which indicate the corresponding positions of the low-resolution tomographic images displayed in the display areas 406 and 407, on the fundus image, which is a planar image of the fundus generated from a plurality of low-resolution tomographic images, to be displayed in the display area 405. Furthermore, the planar image of the fundus does not necessarily need to be a two-dimensional image, but can be a three-dimensional image (for example, an image displayed in the display area 405 illustrated in Fig. 4) enabling checking a plane (surface) of the fundus.

As described above, according to the second embodiment, the acquisition position of a high-resolution tomographic image can be displayed in an easily understandable manner.

In a third embodiment, an image generating apparatus which combines a high-resolution OCT image captured by a measurement system having a high resolution power in the retina transverse direction with an ordinary OCT image captured by a measurement system having a general resolution power is described. More specifically, processing for extracting a high-resolution region limited in the retina depth direction by being captured with a high NA so as to attain high resolution power and combining the extracted high-resolution region with an ordinary OCT image is described.

### <Configuration of Image Generating Apparatus>

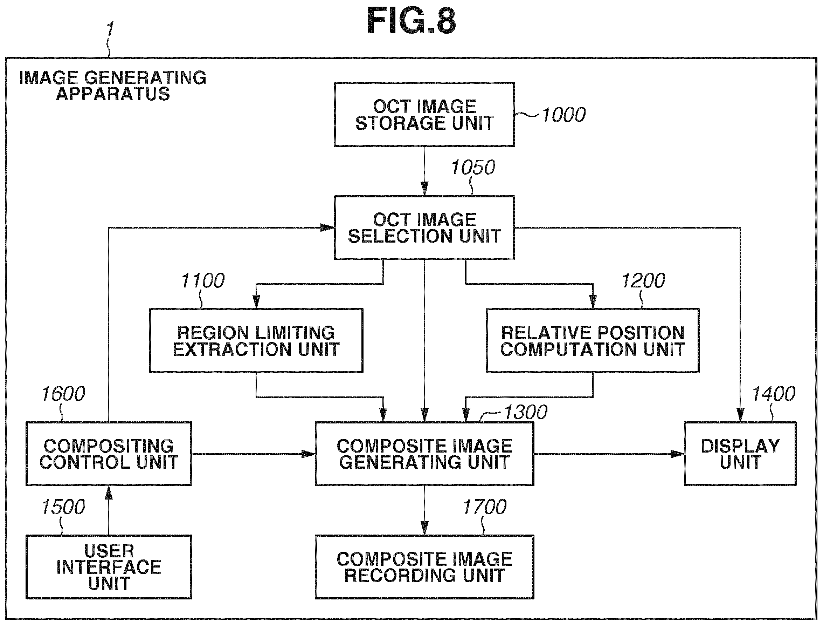

Fig. 8 illustrates a functional configuration of the image generating apparatus 1 according to the third embodiment. Referring to Fig. 8, a plurality of OCT images having respective different resolutions in the retina transverse direction captured by OCT imaging systems having respective different numerical apertures (NAs) is stored in an OCT image storage unit 1000. Herein, a case is described below in which a high-resolution OCT image captured with a measurement beam of 6 mm in diameter with respect to an eye to be examined (which, in an embodiment, corresponds to a first tomographic image with a first resolution) and an ordinary OCT image captured with a measurement beam of 1 mm in diameter (which, in an embodiment, corresponds to a second tomographic image with a second resolution) are stored. Moreover, besides the images, various pieces of accompanying information about the respective images are stored in the OCT image storage unit 1000. The accompanying information includes, for example, information about resolution, information about the right and left eyes, angle-of-view information about an image, reference position information about a reference position on the captured retina image, and an image ID for distinguishing images captured under the same imaging condition. The beam diameter of a measurement beam is used as the information about resolution. Instead, an NA value or a resolution itself can also be used. The reference position information includes information about the presentation position of a fixation target used for image capturing by an OCT imaging apparatus and information about the position scanned with a measurement beam, and the image ID is, for example, a serial number.

An OCT image selection unit 1050 selects an ordinary OCT image targeted for image compositing corresponding to an instruction from the user from among a plurality of OCT images stored in the OCT image storage unit 1000. Moreover, the OCT image selection unit 1050 automatically selects a high-resolution OCT image the imaging region of which overlaps that of the selected ordinary OCT image, using the accompanying information.

A region limiting extraction unit 1100 determines a high-resolution region by performing analysis of resolution on a high-resolution OCT image of the images selected by the OCT image selection unit 1050. Then, the region limiting extraction unit 1100 generates an image of the extracted high-resolution region and transmits the generated image to a composite image generating unit 1300. A relative position computation unit 1200 obtains, by computation, a relative position between the ordinary OCT image and the high-resolution OCT image selected by the OCT image selection unit 1050 and transmits a result of the computation to the composite image generating unit 1300. The relative position is equivalent to the corresponding position of the high-resolution OCT image (the position of the imaging region) on the ordinary OCT image.

The composite image generating unit 1300 generates a composite image based on information about the relative position received from the relative position computation unit 1200. Herein, while the ordinary OCT image selected by the OCT image selection unit 1050 is directly used as an ordinary OCT image, the image received from the region limiting extraction unit 1100 is used as a high-resolution OCT image.

A display unit 1400 presents the generated composite image to the user. A user interface unit 1500 has functions to select a composite image to be displayed and to receive an instruction for a compositing method. The received user instruction is transmitted to a compositing control unit 1600. The compositing control unit 1600 decodes the user instruction into a command or parameter to control OCT image selection by the OCT image selection unit 1050 and image compositing by the composite image generating unit 1300.

When an instruction for storing a composite image is received, a composite image recording unit 1700 records a composite image generated by the composite image generating unit 1300 according to a storage command received via the user interface unit 1500 and the compositing control unit 1600.

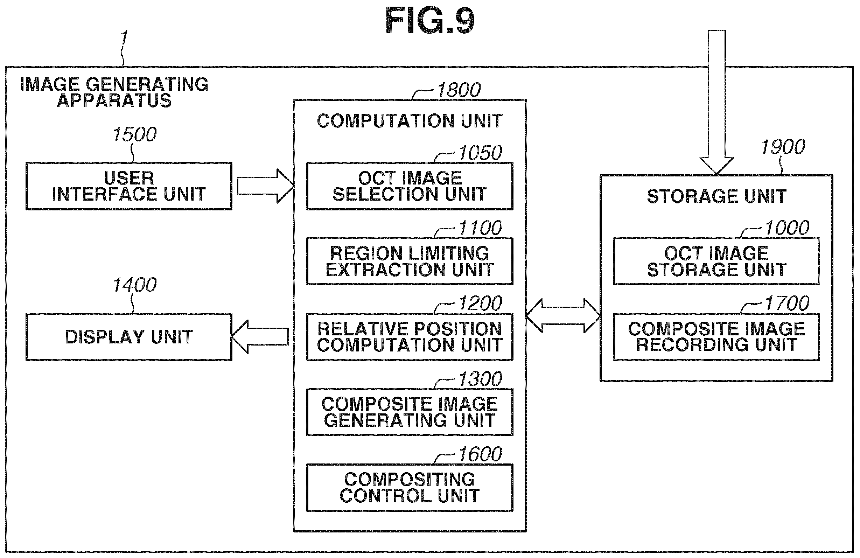

Fig. 9 illustrates a hardware configuration of the image generating apparatus 1 according to the third embodiment. Herein, the image generating apparatus 1 is implemented by a personal computer, and a computation unit 1800 is a central processing unit (CPU). The OCT image selection unit 1050, the region limiting extraction unit 1100, the relative position computation unit 1200, the composite image generating unit 1300, and the compositing control unit 1600 are implemented by the computation unit 1800 executing a previously-stored program. A storage unit 1900 is, for example, a hard disk device, and the OCT image storage unit 1000 and the composite image recording unit 1700 correspond to the storage unit 1900. Furthermore, an OCT image is acquired from a database server, a storage device, or an imaging apparatus located outside the image generating apparatus 1 with the use of a local area network (LAN) or a universal serial bus (USB), which is a general-purpose interface for personal computers, and the acquired OCT image is stored in the OCT image storage unit 1000. The display unit 1400 is a display device, and the user interface unit 1500 is configured with a mouse and a keyboard.

### <Procedure of Image Compositing Processing>

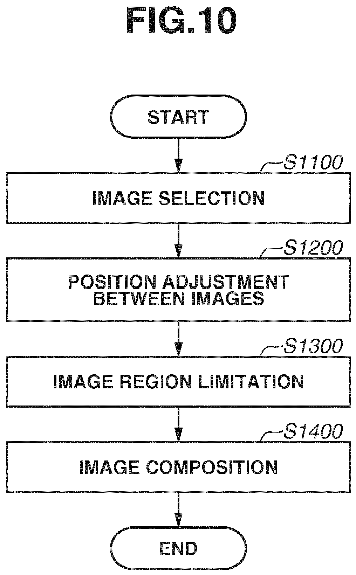

Next, a processing procedure for performing image compositing while limiting a region of a high-resolution OCT image in the image generating apparatus 1 according to the third embodiment is described with reference to the flowchart of Fig. 10.

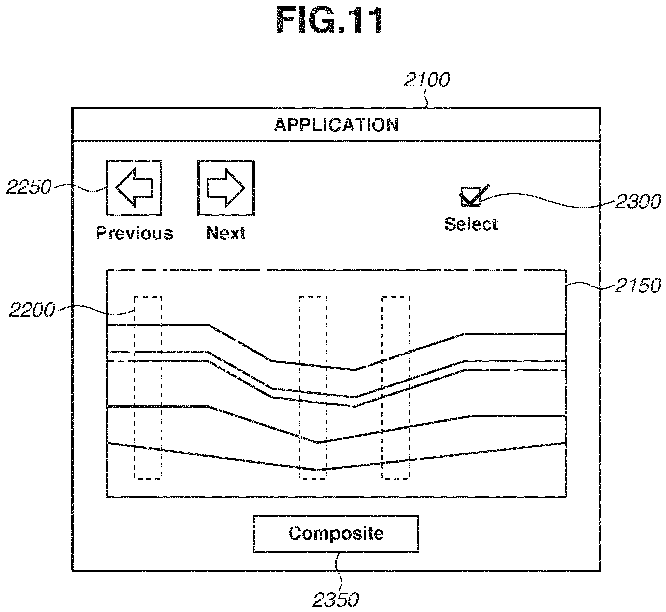

In step S1100, which is a selection process for an image targeted for image compositing, the OCT image selection unit 1050 acquires a list of ordinary OCT images from the OCT image storage unit 1000 and displays the acquired list on the display unit 1400. At this time, the OCT image selection unit 1050 also simultaneously acquires a list of high-resolution OCT images, and draws and displays a frame border on a region over which a high-resolution OCT image laps with respect to each ordinary OCT image using right-and-left eye information, angle-of-view information, and reference position information, each of which is information accompanying each image. Fig. 11 illustrates an OCT image selection screen displayed on the display unit 1400. In a main window 2100 of a program that is being executed on the computer, one ordinary OCT image 2150 is displayed in step S1100. In a case where there is a region over which a high-resolution OCT image laps on the ordinary OCT image 2150 that is being displayed, a frame border 2200 is displayed at the region. In a case where there is a plurality of regions over each of which a high-resolution OCT image laps, a plurality of frame borders is displayed as illustrated in Fig. 11. To perform image compositing with respect to an OCT image that is being displayed, the user checks a checkbox 2300. In a case where there is another ordinary OCT image, the user makes a shift to a next image (or makes a return to a previous image) using an image selection button 2250 and repeats similar processing. Upon completion of selection of an image targeted for image compositing, the user presses an execution button 2350, so that the processing proceeds to preprocessing for image compositing.

In step S1200, which is a position adjustment process between an ordinary OCT image and a high-resolution OCT image, the image generating apparatus 1 sequentially performs position adjustment with respect to all of the combinations of an ordinary OCT image and a high-resolution OCT image from which to generate a composite image.

The image generating apparatus 1 determines an initial positional relationship of images targeted for position adjustment based on reference position information, which is accompanying information about images, calculates a normalized mutual correlation using pixel values of a region at which two images overlap each other, and calculates a correlation coefficient. Next, the image generating apparatus 1 similarly calculates each correlation coefficient while gradually shifting a positional relationship between two images, and obtains a positional relationship according to which the correlation value becomes maximum, thus performing position adjustment between images. In the third embodiment, with respect to a high-resolution OCT image, except for a case where there is an inappropriate region due to, for example, an artifact arising during image capturing or a failure of partial image capturing, the image generating apparatus 1 calculates a correlation coefficient using the entire image without performing region limitation. However, for example, in a case where the NA is relatively large and a tomographic image is obtainable only in the vicinity of the focus position, the image generating apparatus 1 can in some cases previously limit a region in such a way as not to hinder calculation of a correlation coefficient. Furthermore, while, in the third embodiment, a normalized mutual correlation is used, besides, a method for general pattern patching, such as the sum of absolute differences (SAD) (the sum of absolute values of differences of luminance values) or the SSD (the sum of squared differences) (the sum of squares of differences of luminance values), using the similar pixel values can also be used. While limiting a search range to some extent enables making a fast calculation, the entire region can also be searched.

In step S1300, which is a limitation process for a display region with respect to a high-resolution OCT image, the region limiting extraction unit 1100 selects high-resolution OCT images selected by the OCT image selection unit 1050 as image compositing targets one by one in order, determines a portion high in resolution in the planar direction of the fundus (the transverse direction of the image) with respect to each selected image, extracts a region of the determined portion, and sends the extracted region to the composite image generating unit 1300.

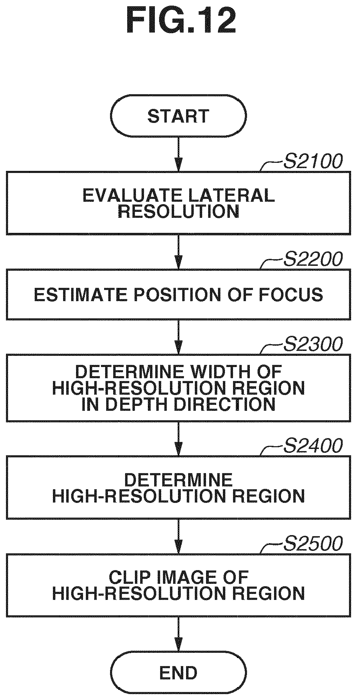

Fig. 12 is a flowchart illustrating details of high-resolution region extraction.

In step S2100, the image generating apparatus 1 evaluates a resolution in the transverse direction of an image corresponding to the retina horizontal direction in an OCT image. More specifically, the image generating apparatus 1 extracts a row of horizontally side-by-side pixels having the same vertical direction position of an image, and performs Fourier transform in the transverse direction using the extracted pixel row. The image generating apparatus 1 performs weighting on the obtained frequency spectral in such a manner that a larger weight is added to a higher frequency portion, integrates the weighted frequency spectral, and sets a result of integration as an evaluation value of resolution in the transverse direction. The image generating apparatus 1 performs the above computation on all of the rows of horizontally side-by-side pixels while changing the vertical direction position, thus obtaining a resolution evaluation value in the transverse direction of each row.

While, in the third embodiment, a resolution is directly used as an evaluation index, since a high resolution makes it easy to distinguish between light and dark, an image quality index, such as contrast, can also be used.

In step S2200, the image generating apparatus 1 estimates the depth position of focus during OCT image capturing using the resolution evaluation values obtained in step S2100. The image generating apparatus 1 plots the obtained resolution evaluation values with respect to the vertical direction position of the OCT image, applies equiangular linear fitting to the plotted values to estimate a position in which the resolution becomes highest, and then sets the estimated position as the depth position of focus.

In step S2300, which is a process for determining the width of a high-resolution region in the retina depth direction of an image centering on the depth position of focus obtained in step S2200, the image generating apparatus 1 obtains the width of a region (the width in the height direction of an image) having a higher resolution in the transverse direction than that of an ordinary OCT image targeted for image compositing.

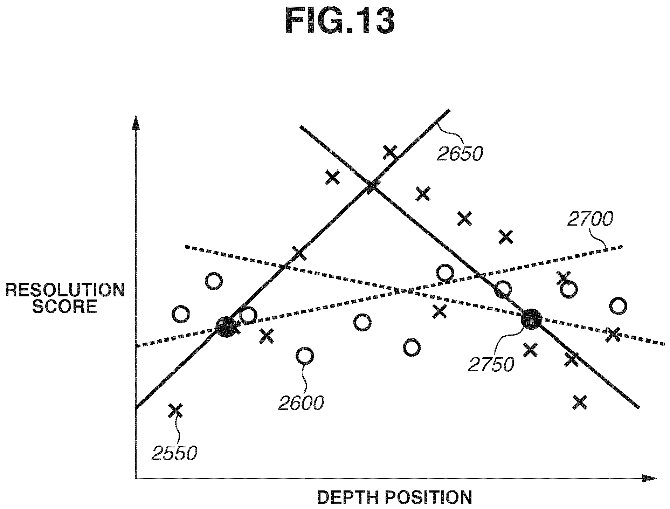

The image generating apparatus 1 extracts only a region over which the selected high-resolution OCT image laps from the ordinary OCT image targeted for image compositing using the positional relationship obtained in step S1200. Then, the image generating apparatus 1 also performs up to the equiangular linear fitting on the extracted ordinary OCT image in a similar way to that in steps S2100 and S2200. Fig. 13 illustrates an example in which the obtained conformal straight lines of an ordinary OCT image and those of a high-resolution OCT image are displayed while lapping over each other. In Fig. 13, a position 2550 indicated by a mark "×" is a resolution evaluation value of the high-resolution OCT image at each depth position (in an embodiment, an example of an image feature), and a position 2600 indicated by a mark "○" is a resolution evaluation value of the ordinary OCT image at each depth position. A straight line 2650 and a dashed line 2700 represent respective results of the equiangular linear fitting. The image generating apparatus 1 obtains two points of positions 2750 which are intersection points between the straight line 2650 and the dashed line 2700, and determines a distance between the two points, i.e., a width in the depth direction of the OCT image, as the width of a high-resolution region.

In step 52400, the image generating apparatus 1 determines, as a high-resolution region, a depth region determined by the width obtained in step S2300 centering on the focus depth position obtained in step S2200. Furthermore, the distance between the two intersection points 2750 of the equiangular linear fitting obtained in step S2300 can be directly determined as a high-resolution region.

In step S2500, the image generating apparatus 1 clips only an image of the high-resolution region in the retina depth direction determined with respect to the selected high-resolution OCT image, thus generating a region limitation image.

In this way, in step S1300, the image generating apparatus 1 determines a region high in resolution in the planar direction of the fundus from the high-resolution OCT image and then clips an image of the determined region.

While, in the third embodiment, the equiangular linear fitting and a result of comparison with an ordinary OCT image are used in steps S2200 and S2300, a reference value for resolution evaluation values can be previously provided and a region in which the resolution evaluation value exceeds the reference value can be determined as a high-resolution region.

In step S1400, the image generating apparatus 1 combines a high-resolution OCT image with an ordinary OCT image based on the results obtained in steps S1200 and S1300 in such a manner that the high-resolution OCT image laps on the ordinary OCT image.

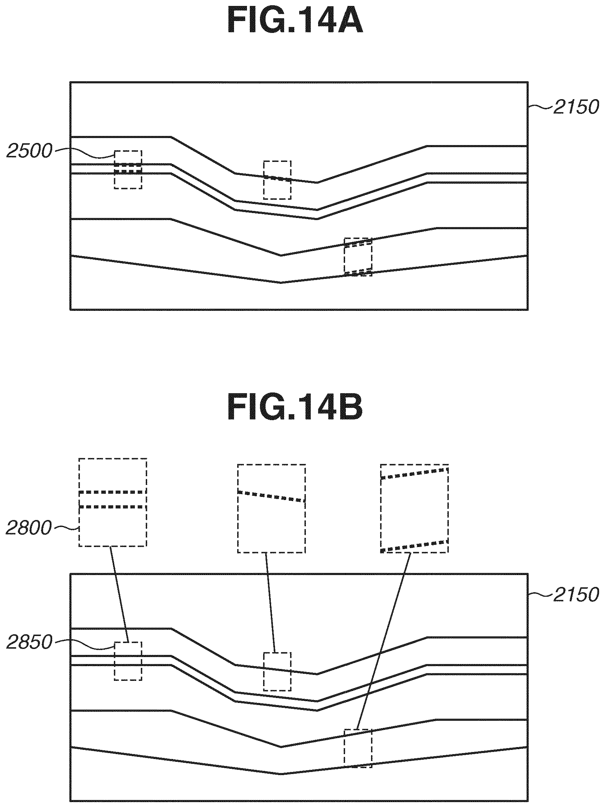

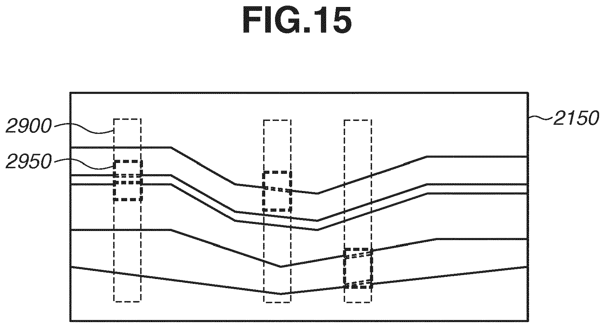

More specifically, the composite image generating unit 1300 combines a high-resolution OCT image, which has been limited to the image of the high-resolution region obtained in step S1300, at the position obtained by using the entire high-resolution OCT image in step S1200. The method for image compositing includes two patterns as illustrated in Figs. 14A and 14B. One of the two patterns is a pattern of directly pasting a region-limited high-resolution OCT image 2500 on an ordinary OCT image 2150, as illustrated in Fig. 14A. The other is a pattern of displaying only a frame 2850 on the ordinary OCT image 2150 and displaying a high-resolution OCT image in a separate frame 2800 in association with the frame 2850, as illustrated in Fig. 14B. Any one of the two patterns can be set according to the user's selection. Furthermore, the display manner illustrated in Fig. 14A can be switched to a display manner illustrated in Fig. 15 according to the user's designation. In Fig. 15, a frame 2900 represents the entirety of a high-resolution OCT image, and a high-resolution region is encompassed by a frame 2950 to expressly provide the position thereof to the user.

Furthermore, to store a composite image which is being displayed on the display unit 1400, the user can perform an input operation on the user interface unit 1500 to issue an instruction to store the composite image in the composite image recording unit 1700, so that the composite image can be stored based on the instruction.

As described above, an image of a high-resolution region is extracted from an OCT image captured with a high NA and including a high-resolution region and a low-resolution region and is then combined with an ordinary OCT image. This enables checking a necessary region with a high-resolution image and also checking the position of an image in the fundus.

In a fourth embodiment, in an image generating apparatus accompanied by OCT with an adaptive optical function, processing for extracting an image of a high-resolution region from an AO-OCT image with a high NA based on parameters obtained from the adaptive optical function and combining the extracted image with an ordinary OCT image is described.

### <Configuration of Image Generating Apparatus>

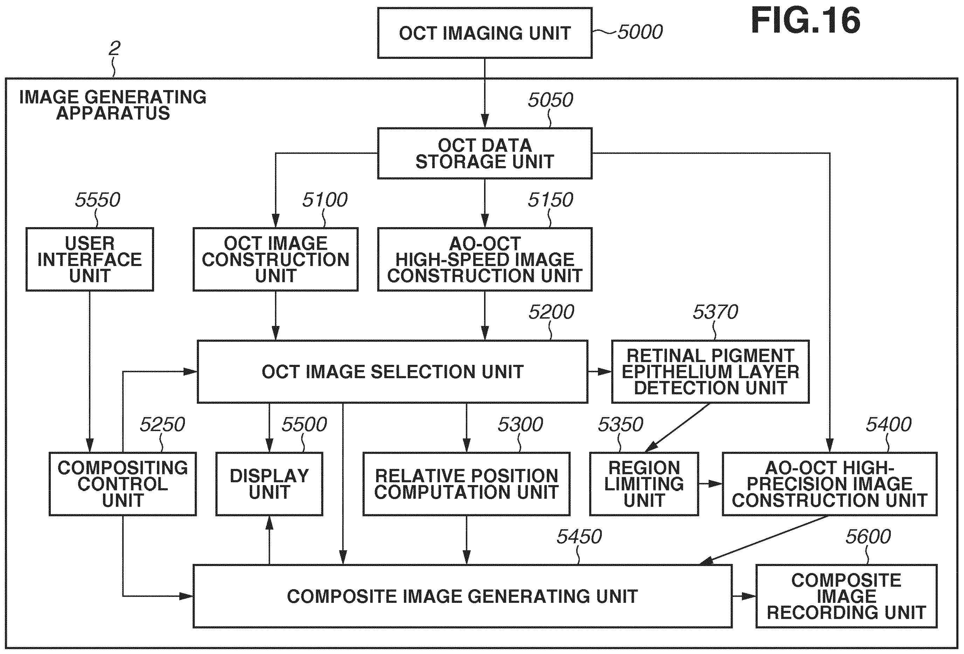

Fig. 16 illustrates a functional configuration of an image generating apparatus 2 according to the fourth embodiment.

An OCT imaging unit 5000, which accompanies the image generating apparatus 2, has two types of modes, an ordinary OCT imaging mode for performing image capturing with a measurement beam of 1 mm in diameter with respect to an eye to be examined and a high-resolution OCT imaging mode for performing image capturing with a measurement beam of 4 mm in diameter while correcting aberrations of an eye to be examined with an adaptive optical function. The captured OCT image is stored in an OCT data storage unit 5050 included in the image generating apparatus 2 while remaining in the form of RAW data, which is not yet reconstructed into a tomographic image. Here, accompanying information about an OCT image is also stored together with the RAW data. In the fourth embodiment, the accompanying information includes, in addition to various pieces of information mentioned in the third embodiment, relative position information about the focus position relative to a retinal pigment epithelium (RPE) layer as information for referring to the focus position used to capture a high-resolution OCT image in the high-resolution OCT imaging mode.

An OCT image construction unit 5100 performs reconstruction processing on RAW data of an ordinary OCT image captured in the ordinary OCT imaging mode to convert the RAW data into a tomographic image. An AO-OCT high-speed image construction unit 5150 does not convert all of RAW data of a high-resolution OCT image captured in the high-resolution OCT imaging mode, but performs reconstruction while thinning out the number of pixels in the retina planar direction of a tomographic image, thus attaining high-speed processing. In the fourth embodiment, an ordinary OCT image is captured at a pixel density (pixel pitch) of 5 µm/pixel with respect to the B-scan direction, which is the retina planar direction, and a high-resolution OCT image is captured at a pixel density of 1 µm/pixel. Since a tomographic image obtained by reconstruction performed by the AO-OCT high-speed image construction unit 5150 is used for position adjustment with an ordinary OCT image, in the fourth embodiment, the number of pixels in the B-scan direction is thinned out to 1/5 so as to also cope with pixel density adjustment with an ordinary OCT image. Furthermore, with regard to the A-scan direction, which is the retina thickness direction, the same processing method is used for the OCT image construction unit 5100 and the AO-OCT high-speed image construction unit 5150, and the angle of view in the A-scan direction is the same between an ordinary OCT image and a high-resolution OCT image. Since, with respect to the retina depth direction, image visualization is performed without region limitation, a region at which images overlap each other in the subsequent relative position computation is made wide so as to facilitate the occurrence of a correlation peak.

An OCT image selection unit 5200 selects an ordinary OCT image targeted for image compositing according to an instruction from the user from among a plurality of ordinary OCT images and a plurality of high-resolution OCT images which have been reconstructed by the OCT image construction unit 5100 and the AO-OCT high-speed image construction unit 5150. Moreover, the OCT image selection unit 5200 automatically selects a high-resolution OCT image the imaging region of which overlaps that of the selected ordinary OCT image, using the accompanying information.

A relative position computation unit 5300 obtains, by computation, a relative position between the ordinary OCT image and the high-resolution OCT image selected by the OCT image selection unit 5200 and transmits a result of the computation to a composite image generating unit 5450.

A retinal pigment epithelium layer detection unit 5370 detects a retinal pigment epithelium (RPE) layer, which is a strong reflecting layer, by image processing in each of the high-resolution OCT images selected by the OCT image selection unit 5200, and obtains the position thereof on the image.

A region limiting unit 5350 determines a high-resolution region based on the following three pieces of information in each of the high-resolution OCT images selected by the OCT image selection unit 5200. The three pieces of information include information about a resolution power, which is accompanying information about an image, relative position information about the focus position, and position information about the RPE obtained by the retinal pigment epithelium layer detection unit 5370. Information about the determined high-resolution region is passed to an AO-OCT high-precision image construction unit 5400. The AO-OCT high-precision image construction unit 5400 reads RAW data from the OCT data storage unit 5050, and converts all of the pixels in the B-scan direction into a tomographic image without thinning out the pixels. However, with respect to the A-scan direction, the AO-OCT high-precision image construction unit 5400 converts only the pixels in the high-resolution region passed from the AO-OCT high-speed image construction unit 5150 into a tomographic image, thus attaining high-speed processing. The reconstructed tomographic image is sent to a composite image generating unit 5450.

The composite image generating unit 5450 generates a composite image based on the relative position information received from the relative position computation unit 5300. Herein, with respect to an ordinary OCT image, the composite image generating unit 5450 directly uses an image selected by the OCT image selection unit 5200, but, with respect to a high-resolution OCT image, the composite image generating unit 5450 uses an image limited to a high-resolution region reconstructed by the AO-OCT high-precision image construction unit 5400.

The functions of a compositing control unit 5250, a display unit 5500, a user interface unit 5550, and a composite image recording unit 5600 are similar to those in the third embodiment, and are, therefore, omitted from description.

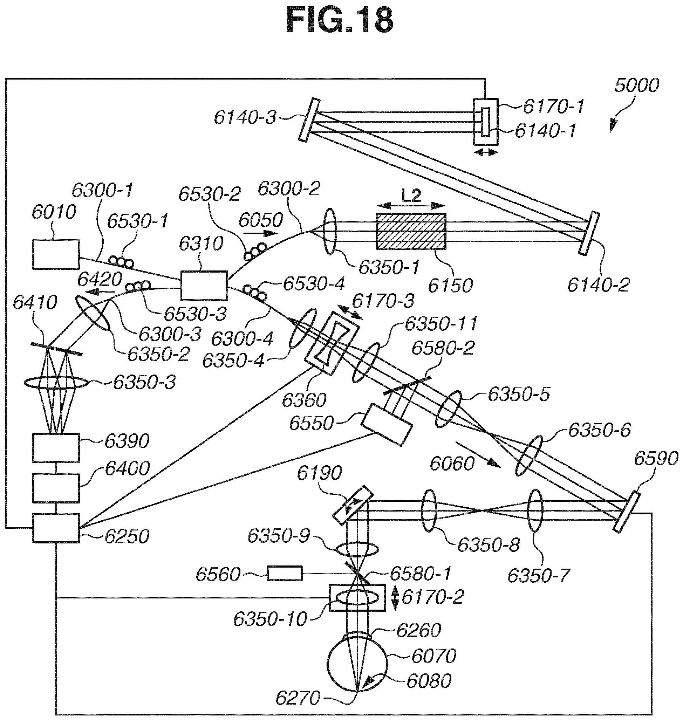

Fig. 18 illustrates a hardware configuration of the image generating apparatus 2 according to the fourth embodiment. The image generating apparatus 2 is implemented by a personal computer and is connected to the OCT imaging unit 5000 via a USB, and OCT data obtained by image capturing is transferred to and stored in a storage unit 5900. The description of the other units is similar to that in the third embodiment, and is, therefore, omitted.

### <Description of OCT Imaging Unit>

The OCT imaging unit 5000 is described.

In the fourth embodiment, the OCT imaging unit 5000 totally configures a Michelson interferometer as illustrated in Fig. 18.

Referring to Fig. 18, light emitted from a light source 6010 is split into reference light 6050 and measurement light 6060 at ratios of 90:10 via an optical fiber 6300-1 and a photo coupler 6310.

The measurement light 6060 is guided to an eye to be examined 6070, which is an observation target, via, for example, an optical fiber 6300-4, a deformable mirror 6590, and an XY scanner 6190. A part of the measurement light 6060 is reflected or scattered by the eye to be examined 6070 and is made to become return light 6080, which is then returned and is combined with the reference light 6050 by the photo coupler 6310.

After being combined with each other, the reference light 6050 and the return light 6080 are spectrally dispersed by a transmission-type grating 6410 by wavelengths and are then made incident on a line camera 6390. The line camera 6390 converts a light intensity into a voltage by positions (wavelengths), thus generating a tomographic image of the eye to be examined 6070 using such voltage signals.

While, in the fourth embodiment, the entire optical system is configured by using a refracting optical system mainly using lenses, it can be configured by using a reflective optical system using spherical mirrors instead of lenses.

Next, the light source 6010 and its periphery are described. The light source 6010 is a super luminescent diode (SLD), which is a typical low-coherence light source. Its wavelength is 830 nm, and its bandwidth is 50 nm. Herein, the bandwidth has an effect on a resolution in the optical axis direction of a tomographic image to be obtained, and is, therefore, an important parameter. Furthermore, with regard to the type of a light source, while an SLD is selected herein, the light source only needs to emit low coherence light and, for example, amplified spontaneous emission (ASE) can also be used. Moreover, with regard to wavelengths, in view of measurement of the eye, near infrared light is suitable. Additionally, since wavelengths have an effect on a resolution in the transverse direction of a tomographic image to be obtained, a wavelength as short as possible is desirable, and the wavelength is 830 nm herein. Depending on a measurement site of the observation target, another wavelength can be selected.

Next, an optical path for the reference light 6050 is described. The reference light 6050, which is obtained by splitting performed by the photo coupler 6310, is guided to a lens 6350-1 via a single mode fiber 6300-2 and is adjusted in such a way as to become parallel light with a beam diameter of 2 mm. Next, the reference light 6050 passes through a dispersion-compensating glass 6150 and is then guided to a mirror 6140-1, which is a reference mirror, via mirrors 6140-2 and 6140-3. Next, the reference light 6050 is reflected by the mirror 6140-1 and is then re-guided to the photo coupler 6310. Herein, the dispersion-compensating glass 6150, through which the reference light 6050 passes, is arranged to compensate for dispersion of the measurement light 6060 reciprocating through the eye to be examined 6070 and lenses 6350-4 to 6350-11 and 6360 with respect to the reference light 6050.

The dispersion-compensating glass 6150 has a length L2, in which L2 = 50 mm is set. Furthermore, an electrically-driven stage 6170-1 is able to move in directions indicated by arrows in Fig. 18, and is thus able to adjust and control the optical path length of the reference light 6050. The electrically-driven stage 6170-1 is controlled by a personal computer 6250.

Next, an optical path for the measurement light 6060 is described.

The measurement light 6060, which is obtained by splitting performed by the photo coupler 6310, is guided to a lens 6350-4 via a single mode fiber 6300-4 and is adjusted in such a way as to become parallel light with a beam diameter of 1 mm. After that, the measurement light 6060 passes through a beam expander, which includes the lenses 6360 and 6350-11, in such a manner that the beam diameter thereof is expanded to one time to four times. This variation of magnification is implemented by driving the lens 6360 with an electrically-driven stage 6170-3 in directions indicated by arrows in Fig. 18. The electrically-driven stage 6170-3 is controlled by the personal computer 6250, and the magnification of the beam expander is set to one time in the ordinary OCT imaging mode and to four times in the high-resolution OCT imaging mode, thus bringing about beam diameters of 1 mm and 4 mm, respectively.

Next, the measurement light 6060 passes through a beam splitter 6580-2 and lenses 6350-5 and 6350-6 and is then made incident on the deformable mirror 6590. Here, the deformable mirror 6590 is a mirror device which freely transforms the mirror shape to correct aberrations of the measurement light 6060 and the return light 6080 based on aberrations detected by a wavefront sensor 6550. While, herein, a deformable mirror is used as a device which corrects aberrations, the device only needs to correct aberrations, so that, for example, a spatial light phase modulator using a liquid crystal can also be used.

Next, the measurement light 6060 passes through lenses 6350-7 and 6350-8 and is then made incident on a mirror of the XY scanner 6190. While, here, for ease of description, one mirror is included in the XY scanner 6190, actually, two mirrors, a mirror for X-scan and a mirror for Y-scan, are juxtaposed to each other to perform raster scan on the retina 6270 in directions perpendicular to the optical axis. Furthermore, the center of the measurement light 6060 is adjusted in such a way as to coincide with the rotation center of the mirror of the XY scanner 6190.

Lenses 6350-9 and 6350-10 configure an optical system used to perform scanning on the retina 6270 and have a role to scan the retina 6270 using the measurement light 6060 with the vicinity of the cornea 6260 used as a fulcrum point. Here, the focal lengths of the lenses 6350-9 and 6350-10 are 50 mm and 40 mm, respectively.

Furthermore, an electrically-driven stage 6170-2 is able to move in directions indicated by arrows in Fig. 18, and is thus able to adjust and control the position of a lens 6350-10 included therein. This enables becoming compatible with the diopter of the eye to be examined 6070.

The electrically-driven stage 6170-2 is configured to be controlled by the personal computer 6250.

Furthermore, a beam splitter 6580-1 reflects visible light and projects a pattern displayed on a fixation target 6560 onto the retina of the eye to be examined 6070, so that the fixation direction of the eye to be examined 6070 can be designated and a region of the retina subjected to OCT image capturing can be varied. An organic EL panel is used as the fixation target 6560.

After entering the eye to be examined 6070, the measurement light 6060 becomes return light 6080 due to reflection or scattering from the retinal 6270, and the return light 6080 is then re-guided to the photo coupler 6310. The above-mentioned reference light 6050 and the return light 6080 are combined by the photo coupler 6310 and are then split at ratios of 90:10. Then, the combined light 6420 is spectrally dispersed by the transmission-type grating 6410 by wavelengths and is then condensed at a lens 6350-3, so that intensities of the light are converted by the line camera 6390 into voltages by positions (wavelengths). More specifically, interference fringes of spectral regions on the wavelength axis are observed on the line camera 6390. The obtained voltage signal group is converted into digital values by a frame grabber 6400, and data processing is performed on the digital values by the personal computer 6250 to form a tomographic image.

Furthermore, a part of the return light 6080, which is obtained by splitting performed by the beam splitter 6580-2, is made incident on the wavefront sensor 6550, so that aberrations of the return light 6080 are measured. The wavefront sensor 6550 is electrically connected to the personal computer 6250. The measured aberrations are expressed using a Zernike polynomial by the personal computer 6250, and represent the aberrations of the eye to be examined 6070.

Moreover, with regard to components of defocus in the Zernike polynomial, the position of the lens 6350-10 is controlled by using the electrically-driven stage 6170-2 so as to correct the diopter of the eye to be examined 6070. With regard to components other than the components of defocus, the surface shape of the deformable mirror 6590 is controlled to make a correction, so that a tomographic image with a higher transverse resolution can be acquired. Additionally, to focus the measurement light 6060 at a predetermined depth position of the retina 6270, the surface shape of the deformable mirror 6590 is controlled to apply offset to a defocus component.

Here, the lenses 6350-5 to 6350-10 are arranged in such a manner that the cornea 6260, the XY scanner 6190, the wavefront sensor 6550, and the deformable mirror 6590 are optically conjugate with each other, thus enabling the wavefront sensor 6550 to measure aberrations of the eye to be examined 6070.

### <Derivation of Focus Position>

A method of obtaining the focus position of measurement light, which is information accompanying an image, when the OCT imaging unit 5000 performs image capturing in the high-resolution OCT imaging mode is described.

The OCT imaging unit 5000 performs aberration correction by controlling the deformable mirror 6590 while observing aberrations measured by the wavefront sensor 6550. Furthermore, a defocus item in the Zernike polynomial of the aberrations is corrected by adjusting and controlling the position of the lens 6350-10. When these corrections are completed, the measurement light 6060 enters a state of being focused on the RPE, which is a high reflecting layer of the retina.

The OCT imaging unit 5000 previously stores a correction value for a defocus item in the Zernike polynomial which is required to correct aberrations of the standard eye with a refractive power of 60 diopter (D), i.e., the position of the lens 6350-10 obtained by driving of the electrically-driven stage 6170-2. The refractive power of the eye to be examined is determined by comparing the stored correction value with a correction amount for the defocus item which has been actually used to correct aberrations of the eye to be examined, i.e., the actual driving position of the electrically-driven stage 6170-2. Next, a high-resolution OCT image is captured while changing the focus position in the retina depth direction by increasing and decreasing the defocus item of the correction amount with the deformable mirror 6590. At this time, the relative position of the focus position to the RPE is calculated based on the measured refractive power value of the eye to be examined and the increase and decrease value of the defocus item applied to the deformable mirror 6590 to change the focus position. This becomes relative position information about the focus position accompanying a high-resolution OCT image.

### <Procedure of Image Compositing Processing>

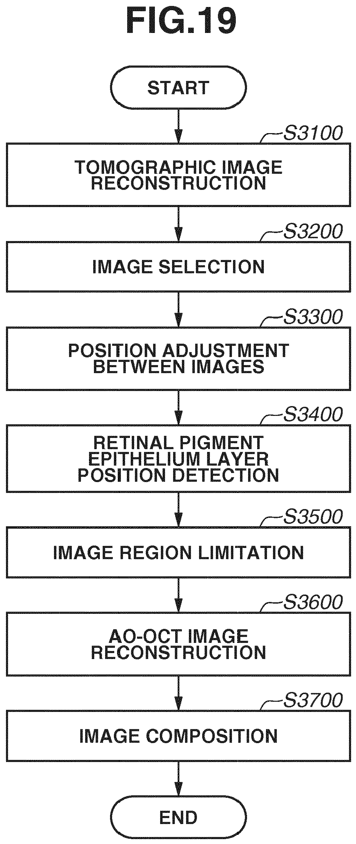

Next, in the image generating apparatus 2 according to the fourth embodiment, a processing procedure for extracting an image of a designated high-resolution region in the vicinity of the focus from a high-resolution OCT image and combining the extracted image with an ordinary OCT image is described with reference to the flowchart of Fig. 19.

In step S3100, which is a process of reconstructing a tomographic image from RAW data of OCT stored in the OCT data storage unit 5050, when processing is started, the OCT image construction unit 5100 and the AO-OCT high-speed image construction unit 5150 read RAW data of an ordinary OCT image and RAW data of a high-resolution OCT image, respectively, from the OCT data storage unit 5050, and perform reconstruction processing to reconstruct respective tomographic images. At this time, as mentioned above, the AO-OCT high-speed image construction unit 5150 thins out the number of pixels in the B-scan direction to 1/5, thus performing reconstruction processing at high speed. The constructed OCT images are sent to the OCT image selection unit 5200.

Step S3200 is a selection process for images targeted for image compositing, and step S3300 is a position adjustment process between an ordinary OCT image and a high-resolution OCT image. A high-resolution OCT image used in these processes is a tomographic image reconstructed by the AO-OCT high-speed image construction unit 5150, and the pixel density thereof in the B-scan direction is equal to that of an ordinary OCT image and is thus coarse. The other description of these steps is similar to that of steps S1100 and S1200 in the third embodiment, and is, therefore, omitted. Furthermore, in the fourth embodiment, since a result of the position adjustment process in step S3300 is used for image compositing performed in step S3700, the processing order of step S3300 is optional between step S3200 and step S3700, and can be set as background processing of another processing operation.

In step S3400, which is a process of detecting the position of the RPE serving as a reference of the focus position of measurement light for a high-resolution OCT image, the retinal pigment epithelium layer detection unit 5370 detects the RPE with respect to each of high-resolution OCT images reconstructed by the AO-OCT high-speed image construction unit 5150 and selected by the OCT image selection unit 5200. Since the RPE is a high reflecting layer located at the deepest position in an OCT image, the retinal pigment epithelium layer detection unit 5370 detects a position corresponding to the RPE by evaluating the luminance of each pixel with respect to the A-scan. The retinal pigment epithelium layer detection unit 5370 calculates an average value of respective RPE positions of all of the A-scan images configuring a single high-resolution OCT image, sets the calculated average value as RPE position information, and sends the RPE position information to the region limiting unit 5350.

Step S3500 is a process of designating a region in which a high resolution is attained in a high-resolution OCT image, using information about a resolution and relative position information about the focus position, which are accompanying information about an OCT image, and the position information about the RPE obtained in step S3400.

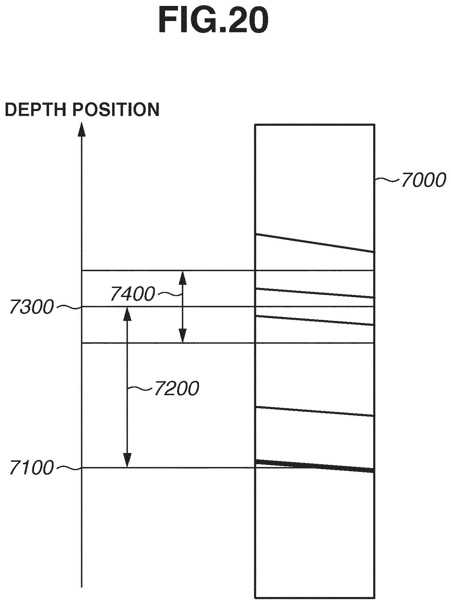

Referring to Fig. 20, in an OCT image 7000 from which an image of a region in a single high-resolution OCT image is extracted, a region indicated by a double-headed arrow 7400 in the retina depth direction corresponds to the depth of focus and is thus a region in which a high resolution is attained. This region is obtained in the following way. The RPE position obtained in step S3400 is a depth position 7100, and a distance 7200 from the RPE to the focus position of measurement light is obtained from the relative position of the focus position to the RPE, which is the accompanying information. With this, the depth position of the focus position 7300 of measurement light is determined. The depth of focus 7400, which is the information about a resolution, is calculated from the beam diameter of 4 mm of measurement light to be approximately 60 µm. The above processing is performed by the region limiting unit 5350, so that a region having the width of the depth of focus centering on the focus position of measurement light, which is a high-resolution region, is designated.

In step S3600, which is a process of reconstructing a high-resolution OCT image in the region determined in step S3500, the AO-OCT high-precision image construction unit 5400 reads out RAW data from the OCT data storage unit 5050 based on the position information about the high-resolution region of each high-resolution OCT image obtained from the region limiting unit 5350, and performs image reconstruction processing only on pixels in the range of the high-resolution region to convert the pixels into a tomographic image. With this, a high-resolution OCT image with only a high-resolution region extracted is constructed. Furthermore, since thinning-out processing in the B-scan direction, which the AO-OCT high-speed image construction unit 5150 has performed in step S3100, is not performed here, an image in the B-scan direction made into a high resolution by the effect of AO is expressed by a sufficient number of pixels.

Step S3700 is a process of combining a high-resolution OCT image with an ordinary OCT image based on the results obtained in steps S3200, S3300, and S3600 in such a manner that the high-resolution OCT image laps on the ordinary OCT image.

The composite image generating unit 5450 combines a high-resolution OCT image obtained in step S3600 with an ordinary OCT image selected in step S3200 according to the relative position obtained in step S3300. A result of image compositing is displayed on the display unit 5500, and the composite image is stored in the composite image recording unit 5600.

As described above, in displaying an OCT image excellent in resolution in the retina planar direction captured by AO-OCT, a high-resolution region limited to the vicinity of the focus of an OCT image captured by AO-OCT is extracted and is then combined with an ordinary OCT image. This enables checking a necessary region with a high-resolution image and also checking the position of an image in the fundus.

While, in the above-described embodiments, image-captured data is acquired by a spectral domain type OCT using a broadband light source with respect to the eye to be examined 118, the present invention is not limited to this, but, for example, a swept-source OCT can also be used.

Furthermore, while, in the above-described embodiments, a case where an object to be examined is the eye is described, the present invention can be applied to an object to be examined other than the eye, such as a skin and an organ. In this case, the present invention takes the form of a medical apparatus, such as an endoscope, other than the ophthalmic imaging apparatus. Accordingly, it is desirable that the present invention be understood as an image acquisition apparatus, an example of which is an ophthalmic imaging apparatus, and an eye to be examined be understood as one form of an object to be examined.

### Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random access memory (RAM), a read-only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to embodiments, it is to be understood that the invention is not limited to the disclosed embodiments.

## Claims

1. A tomographic image acquisition apparatus comprising:
first designation means for designating a first position in which to acquire a first tomographic image in a fundus image of an eye to be examined;
first acquisition means for acquiring the first tomographic image corresponding to the first position;
second designation means for designating a second position in which to acquire a second tomographic image in the first tomographic image, the second tomographic image having a higher resolution than the first tomographic image; and
second acquisition means for acquiring the second tomographic image based on the first position and the second position.

2. The tomographic image acquisition apparatus according to claim 1, wherein the fundus image of the eye to be examined is an image generated by projecting a plurality of first tomographic images in an optical axis direction.

3. The tomographic image acquisition apparatus according to claim 1, further comprising:
splitting means for splitting light from a light source into measurement light and reference light;
control means for adjusting an optical path length difference between the measurement light and the reference light; and
focus means for adjusting a focus position of the measurement light,
wherein the focus means adjusts the focus position to be used to acquire the second tomographic image based on information about the optical path length difference adjusted to acquire the first tomographic image.

4. The tomographic image acquisition apparatus according to claim 3, further comprising correction means for correcting aberrations of return light caused by irradiating the eye to be examined with the measurement light,
wherein the second position is designated as a rectangular region, and
wherein, in a case where a size in a horizontal direction of the designated rectangular region exceeds a size that is able to be corrected by the correction means, the second designation means acquires a plurality of second tomographic images respectively in a plurality of regions into which the rectangular region is divided.

5. The tomographic image acquisition apparatus according to claim 4, further comprising compositing means for combining the acquired plurality of second tomographic images to generate one tomographic image.

6. The tomographic image acquisition apparatus according to claim 5, further comprising position adjustment means for performing position adjustment on the acquired plurality of second tomographic images based on a reference image selected from the acquired plurality of second tomographic images,
wherein the compositing means combines the plurality of second tomographic images subjected to the position adjustment.

7. The tomographic image acquisition apparatus according to claim 4, wherein, in a case where the plurality of regions into which the rectangular region is divided includes a plurality of regions arranged in both vertical and horizontal directions, the second acquisition means prioritizes acquisition in regions arranged in the vertical direction.

8. The tomographic image acquisition apparatus according to claim 3, further comprising change means for changing a beam diameter of the measurement light,
wherein the first acquisition means and the second acquisition means use respective different beam diameters of the measurement light, and the beam diameter used by the second acquisition means is larger than that used by the first acquisition means.

9. A tomographic image acquisition apparatus comprising:
display means having a first display area in which to display a second tomographic image, a second display area in which to display a first tomographic image, and a third display area in which to display a planar image, the second tomographic image having a higher resolution than the first tomographic image; and
control means for displaying a position corresponding to the second tomographic image displayed in the first display area on each of the first tomographic image displayed in the second display area and the planar image displayed in the third display area.

10. The tomographic image acquisition apparatus according to claim 9, wherein the planar image is a planar image of a fundus generated by projecting a plurality of first tomographic images in an optical axis direction.

11. The tomographic image acquisition apparatus according to claim 1, further comprising determination means for determining a range of the second tomographic image based on a resolution evaluation value of each of the first tomographic image and the second tomographic image.

12. A tomographic image acquisition method comprising:
designating a first position in which to acquire a first tomographic image in a fundus image of an eye to be examined;
acquiring the first tomographic image corresponding to the first position;
designating a second position in which to acquire a second tomographic image in the first tomographic image, the second tomographic image having a higher resolution that the first tomographic image; and
acquiring the second tomographic image based on the first position and the second position.

13. A tomographic image acquisition method comprising:
displaying a second tomographic image in a first display area, displaying a first tomographic image in a second display area, and displaying a planar image in a third display area, the second tomographic image having a higher resolution than the first tomographic image; and
displaying a position corresponding to the second tomographic image displayed in the first display area on each of the first tomographic image displayed in the second display area and the planar image displayed in the third display area.

14. A tomographic image acquisition apparatus configured to set a region of a first tomographic image obtained by capturing an image of an eye to be examined using an optical system with a first numerical aperture, the tomographic image acquisition apparatus comprising:
acquisition means for acquiring a second tomographic image obtained by capturing the image using an optical system with a second numerical aperture which is lower than the first numerical aperture;
position adjustment means for performing position adjustment between the first tomographic image and the second tomographic image;
comparison means for making a comparison between an image feature of the first tomographic image and an image feature of the second tomographic image based on a result of the position adjustment; and
setting means for setting a region of the second tomographic image based on a result of the comparison.

15. The tomographic image acquisition apparatus according to claim 14, wherein the image feature is an image resolution.

16. The tomographic image acquisition apparatus according to claim 14, further comprising control means for displaying an image of the region set in the second tomographic image on display means.

17. The tomographic image acquisition apparatus according to claim 16, wherein the control means displays the second tomographic image on the display means, and displays the image of the region set in the second tomographic image at a position corresponding to the region on the displayed second tomographic image.

18. The tomographic image acquisition apparatus according to claim 16, wherein the control means displays the second tomographic image on the display means, and displays information indicating the region set in the second tomographic image at a position corresponding to the region on the displayed second tomographic image and displays the image of the region set in the second tomographic image in association with the information.

19. The tomographic image acquisition apparatus according to claim 14, wherein the comparison means makes a comparison in corresponding image resolution in a transverse direction between the first tomographic image and the second tomographic image.

20. The tomographic image acquisition apparatus according to claim 14, wherein the comparison means performs the comparison while limiting a region subjected to the comparison based on a focus position used to capture the first tomographic image.

21. The tomographic image acquisition apparatus according to claim 14, further comprising an adaptive optical system configured to measure a wavefront of return light from the eye to be examined and correct the measured wavefront,
wherein the first tomographic image is an image captured via the adaptive optical system.

22. A tomographic image acquisition apparatus comprising:
acquisition means for acquiring a first tomographic image with a first resolution and a second tomographic image with a second resolution lower than the first resolution;
detection means for obtaining a relative position of the first tomographic image to the second tomographic image; and
control means for displaying the first tomographic image and the second tomographic image on display means based on the relative position,
wherein the detection means obtains the relative position from a region of the second tomographic image limited based on information about a focus position used to capture the first tomographic image.

23. The tomographic image acquisition apparatus according to claim 22, wherein the control means displays the first tomographic image at the relative position on the second tomographic image in a superimposed manner.

24. The tomographic image acquisition apparatus according to claim 22, wherein the control means displays information indicating the first tomographic image at the relative position on the second tomographic image and displays the first tomographic image in association with the information.

25. A tomographic image acquisition method of setting a region of a first tomographic image obtained by capturing an image of an eye to be examined using an optical system with a first numerical aperture, the tomographic image acquisition method comprising:
acquiring a second tomographic image obtained by capturing the image using an optical system with a second numerical aperture which is lower than the first numerical aperture;
performing position adjustment between the first tomographic image and the second tomographic image;
making a comparison between an image feature of the first tomographic image and an image feature of the second tomographic image based on a result of the position adjustment; and
setting a region of the second tomographic image based on a result of the comparison.

26. A tomographic image acquisition method comprising:
acquiring a first tomographic image with a first resolution and a second tomographic image with a second resolution lower than the first resolution;
obtaining a relative position of the first tomographic image to the second tomographic image; and
displaying the first tomographic image and the second tomographic image on display means based on the relative position,
wherein the relative position is obtained from a region of the second tomographic image limited based on information about a focus position used to capture the first tomographic image.
